# FASCICULE DE BREVET EUROPEEN

(11) **EP 4 262 741 B1**
(45) Date de publication et mention de la délivrance du brevet: **25.06.2025**
(21) Numéro de dépôt: 21848172.9
(22) Date de dépôt: 13.12.2021
(51) Int. Cl.: A61K 8/9789, A61Q 5/00, A61Q 7/00

(54) **UTILISATIONS COSMÉTIQUES D'UN HYDROLYSAT DE TOURTEAU D'HIPPOPHAE RHAMNOIDES**
KOSMETISCHE VERWENDUNGEN EINES HIPPOPHAE-RHAMNOIDES-KUCHENHYDROLYSATS
COSMETIC USES OF A HIPPOPHAE RHAMNOIDES CAKE HYDROLYSATE

(30) Priorité: 15.12.2020 FR 2013229
(43) Date de publication de la demande: 25.10.2023
(73) Titulaire: BASF Beauty Care Solutions France SAS, 69007 Lyon (FR)
(72) Inventeur: DANOUX, Louis, 54425 ESSEY-LES-NANCY (FR); HENRY, Florence, 54425 ESSEY-LES-NANCY (FR); MINE GENSOLLEN, Solène, 54425 ESSEY-LES-NANCY (FR); MOSER, Philippe, 54425 ESSEY-LES-NANCY (FR)
(74) Mandataire: Cabinet Beau de Loménie
(86) Numéro de dépôt international: PCT/FR2021/052285
(87) Numéro de publication internationale: WO 2022/129750

(56) Documents cités:
- EP-A1- 1 759 683
- CN-A- 1 490 036
- CN-A- 108 354 862
- FR-A1- 2 943 255
- FR-A1- 2 951 944
- DATABASE GNPD [online] MINTEL; 17 March 2020 (2020-03-17), ANONYMOUS: "Restorative Hair Serum", XP055848347, retrieved from https://www.gnpd.com/sinatra/recordpage/7446557/ Database accession no. 7446557
- DATABASE GNPD [online] MINTEL; 17 March 2020 (2020-03-17), ANONYMOUS: "Restorative Hair Serum", XP055848347, retrieved from https://www.gnpd.com/sinatra/recordpage/7446557/ Database accession no. 7446557
- KOSKOVAC MARIJANA ET AL: "Sea Buckthorn Oil-A Valuable Source for Cosmeceuticals", COSMETICS, vol. 4, no. 4, 16 December 2017 (2017-12-16), pages 40, XP055848351, DOI: 10.3390/cosmetics4040040

## Description

### Domaine Technique

L'invention est relative à de nouvelles utilisations cosmétiques d'un hydrolysat de tourteau d'*Hippophae rhamnoides.*

### Technique antérieure

Le soin des annexes cutanées telles que les cheveux, les cils, les sourcils, implique un traitement global, c'est à dire un traitement direct de la fibre capillaire ou fibre kératinique mais aussi de la zone de peau correspondante, y inclus les follicules pileux. S'agissant des cheveux, ces follicules pileux sont répartis sur tout le cuir chevelu et sont responsables de la croissance des cheveux. Ces follicules comprennent en effet des cellules d'origine épithéliales prolifératives dans la partie inférieure du follicule, au niveau du bulbe.

En outre, le soin direct de la fibre capillaire implique le traitement des protéines qui la constituent. Les kératines par exemple sont particulièrement sensibles aux endommagements induits par le style de vie urbain, à la fréquence d'utilisation de produits chimiques d'hygiène, aux habitudes de coiffage mais aussi aux conditions environnementales, c'est-à-dire soleil, pollution, sel, vent, variations de conditions climatiques, à l'hygiène de vie en général ainsi que l'âge. Ces protéines vont se dénaturer et rendre les annexes cutanées, en particulier les cheveux moins résistants, moins souples, cassants, mais aussi plus ternes et plus fourchus. Ces annexes perdent leurs propriétés biomécaniques et leurs propriétés de surface.

Une solution est l'apport de protéines exogènes permettant de compenser l'endommagement des protéines capillaires.

L'hydrolyse (chimique, thermique ou enzymatique) est un processus nécessaire toutefois pour fragmenter en faible poids moléculaire les protéines, favoriser leur solubilité dans l'eau et en l'occurrence leur pénétration dans la fibre capillaire, les rendant utilisables dans les formulations cosmétiques. Seules les molécules de faible poids moléculaires peuvent pénétrer dans la fibre, en particulier dans les fibres capillaires endommagées. Ces protéines hydrolysées aident à améliorer le maintien d'hydratation, l'élasticité des fibres capillaires et apportent plus de douceur, de brillance, de rebond et de corps. Elles permettent de renforcer la fibre capillaire depuis l'intérieur.

Les protéines hydrolysées performantes présentent des propriétés de substantivité pour la fibre capillaire (liaisons faibles avec les kératines du cheveu) du fait des charges ioniques et des sites polaires (interactions par des forces de van der Walls). Elles peuvent également former des films protecteurs à la surface de la fibre capillaire.

Des produits à base de protéines hydrolysées existent sur le marché de la cosmétique. Ces protéines sont d'origine végétale ou animale. Les plus courantes sont des protéines hydrolysées de blé, de soie, de kératine, de collagène, d'élastine, de lait, d'amandes. Il existe toutefois un besoin constant d'ingrédients alternatifs performants dans le domaine.

De manière tout à fait inattendue, les inventeurs ont constaté qu'un hydrolysat de tourteau *d'H. rhamnoides* avait la capacité de pénétrer la fibre kératinique, pour en augmenter ses propriétés mécaniques, ainsi que ses propriétés de texture et de surface. Cet hydrolysat s'est avéré capable d'augmenter la croissance des annexes cutanées, lutter contre leur chute, augmenter leur résistance et diminuer leur casse, en particulier celle des cheveux, les rendant plus brillants, plus éclatants et avec du volume. En outre, l'hydrolysat de tourteau d'H. *rhamnoides* possède des propriétés de réparation des annexes cutanées tel qu'il sera démontré dans la suite de la description.

Un avantage de l'hydrolysat selon la présente invention est qu'il s'agit d'un produit issu d'un résidu d'extraction industriel habituellement non valorisé, le développement du produit s'inscrivant ainsi dans une démarche éco-responsable. Un autre avantage encore est qu'il s'agit d'un ingrédient assurant un soin complet, car ayant des effets à la fois sur la fibre kératinique, en particulier le cheveu, mais aussi sur le cuir chevelu et les follicules pileux. En outre, l'hydrolysat selon l'invention peut être facilement produit à l'échelle industrielle. Enfin, l'hydrolyse enzymatique permet de fractionner les protéines du tourteau pour récupérer des peptides de faible poids moléculaire, ceux précisément pouvant pénétrer les fibres kératiniques pour en assurer les effets cosmétiques décrits ici.

L'hydrolysat selon l'invention provient du tourteau de la plante *Hippophae rhamnoides.* Cette plante aussi appelé argousier est connue pour ses usages anciens. Ainsi, en Grèce antique, ses feuilles étaient utilisées pour la nourriture des animaux, en particulier des chevaux pour augmenter la brillance de leur robe. Les grecs utilisaient également les baies de manière thérapeutique pour diminuer la douleur, les maux d'estomac et soigner le scorbut. Des extraits de feuilles ont aussi été utilisés pour traiter l'asthme, les ulcères gastriques, les problèmes de peau (cicatrisation, brûlures) et de poumons. En Chine, le fruit *d'H. rhamnoides* est utilisé pour soulager la toux et transformer le flegme, fortifier l'estomac et soulager les digestions difficiles, activer le sang et dissiper les stases. En Asie centrale, les populations locales utilisaient l'Argousier pour traiter l'hypertension, les désordres digestifs et cutanés. L'huile extraite des baies est utilisée pour ses propriétés anti-inflammatoires et les ulcères gastriques. La décoction des fruits secs est utilisée pour les problèmes de peau.

D'autre part, des extraits *d'H. rhamnoides* existent déjà sur le marché de la cosmétique. Ainsi, un extrait aqueux du coproduit d'extraction au CO₂ supercritique des graines *d'H. rhamnoides* est commercialisé par la Demanderesse sous la dénomination RNAge^{™} pour ses propriétés anti-âges sur la peau et les muqueuses et a été décrit dans la demande de brevet WO2019/069007. L'extrait selon la présente invention est toutefois différent de ce dernier puisqu'il s'agit d'un hydrolysat du coproduit d'extraction au CO₂ supercritique des graines. Les effets cosmétiques décrits dans la présente invention en sont par ailleurs distincts.

Les demandes CN105063139 et CN108065411 divulguent des hydrolysats peptidiques provenant du résidu d'extraction au CO₂ supercritique des graines *d'H. rhamnoides.* Il s'agit d'hydrolysats obtenus par hydrolyse enzymatique. Toutefois, aucune de ces deux demandes ne divulguent les applications cosmétiques ni dermatologiques faisant l'objet de la présente invention.

La demande CN108354862 décrit une composition comprenant entre autre une huile *d'H. rhamnoides,* pour un effet antipelliculaire et apaisant. Toutefois il s'agit d'une huile et non d'un hydrolysat d'une part et les effets cosmétiques décrits dans la présente invention sont distincts de l'effet antipelliculaire de cette demande d'autre part.

La demande FR3031455 décrit plusieurs molécules pouvant être extraites *d'H. rhamnoides* et destinées à être incorporées dans une composition cosmétique notamment en tant qu'agent réparateur, entre autres pour les cheveux. Toutefois, aucune divulgation ni aucune suggestion des effets précis décrits dans la présente invention n'est mentionnée. Par ailleurs, un extrait de la plante *H. rhamnoides* est divulgué sans plus de description du type d'extrait.

La demande FR2943255 décrit l'utilisation d'un extrait d'huile *d'H. rhamnoides* obtenu par extraction au CO₂ supercritique pour stimuler l'activité de l'alpha-5-réductase, pour améliorer la perte de brillance des cheveux. Il s'agit cependant du produit d'extraction au CO₂ supercritique, pas de son coproduit, encore moins de l'hydrolysat de ce dernier. Par ailleurs, les effets cosmétiques décrits dans la présente demande n'y sont ni divulgués ni suggérés.

La demande WO2009125071 décrit plusieurs extraits de plante obtenus à partir de coproduits d'extraction au CO₂ supercritique, lesquels coproduits sont ensuite soumis à extraction hydroalcoolique ou avec un mélange eau et butylène glycol notamment. Un extrait de fruit *d'H. rhamnoides* est divulgué. Toutefois, l'extrait de la présente invention en est distinct puisqu'il s'agit d'un hydrolysat du coproduit d'H. *rhamnoides.* Cette demande ne décrit pas non plus du tout les effets cosmétiques décrits dans la présente invention et en particulier ne décrit aucun effet sur les annexes cutanées. Les documents CN 1 490 036 A et EP 1 759 683 A1 divulguent des traitements pour l'alopécie et la calvitie utilisant des extraits d'*Hippophae rhamnoides.* Le document FR 2 951 944 A1 divulgue un extrait de tourteau de soja.

Ainsi à la connaissance de la demanderesse, aucun art antérieur ne divulgue les utilisations cosmétiques ou pharmaceutiques de l'hydrolysat selon la présente invention. Aucun document seul ou en combinaison ne les suggère non plus.

### Exposé de l'invention

Un premier objet concerne ainsi l'utilisation cosmétique non thérapeutique d'un hydrolysat de tourteau d'*H*. *rhamnoides* pouraugmenter pour augmenter la croissance des annexes cutanées et/ou diminuer leur chute, avantageusement des cheveux; et/ou pour maintenir et/ou augmenter les propriétés biomécaniques et/ou les propriétés de surface et/ou de texture des annexes cutanées, avantageusement des cheveux ; et/ou pour réparer les annexes cutanées abîmées, avantageusement les cheveux.

Un second objet est relatif à l'utilisation cosmétique non thérapeutique de l'hydrolysat dans une composition cosmétique.

Un 3^{ème} objet concerne un procédé de soin cosmétique non thérapeutique comprenant l'application par voie topique de l'hydrolysat selon l'invention ou d'une composition cosmétique le comprenant.

Un 4^{ème} objet est relatif à une méthode de traitement cosmétique et un dernier objet concerne l'hydrolysat selon l'invention pour son utilisation dermatologique ou pharmaceutique.

Un premier objet concerne donc l'utilisation cosmétique non thérapeutique d'un hydrolysat de tourteau d'*H*. *rhamnoides* pour augmenter la croissance des annexes cutanées et/ou diminuer leur chute, avantageusement des cheveux ; et/ou pour maintenir et/ou augmenter les propriétés biomécaniques et/ou les propriétés de surface et/ou de texture des annexes cutanées, avantageusement des cheveux ; et/ou pour réparer les annexes cutanées abîmées, avantageusement les cheveux.

On entend par « utilisation cosmétique » une utilisation non thérapeutique, non pharmaceutique ni dermatologique, c'est-à-dire qui ne nécessite pas de traitement thérapeutique et destinée à des peaux et/ou des annexes cutanées et/ou des muqueuses saines. On entend par « saines » des peaux ou des annexes cutanées ou des muqueuses qualifiées de non pathologiques par un spécialiste du domaine, un dermatologue, c'est à dire qui ne présentent pas d'infection, d'inflammation, de cicatrice, de maladie ou d'affection cutanée telle que candidose, impétigo, psoriasis, eczéma, acné ou dermatite notamment séborrhéique, pellicules, ou plaies ou blessures et/ou autres dermatoses et/ou d'alopécie et/ou de calvitie. Ainsi au sens de l'invention, des annexes cutanées « abîmées » sont des annexes qualifiées de non pathologiques par le spécialiste du domaine. De façon particulière, des annexes cutanées abîmées sont des annexes qui ont perdu leur souplesse et/ou leur élasticité et/ou leur capacité de déformation notamment et qui par conséquent sont déshydratées. Les annexes cutanées « abîmées » perdent leur structure et/ou les propriétés visuelles et/ou biomécaniques. Ainsi, ils deviennent ternes et rugueux, sont moins résistants, deviennent friables, cassants, se dédoublent et/ou se recourbent et sont donc fourchus. Ils sont par ailleurs rugueux et donc moins doux, difficiles à coiffer et à mettre en forme. Cette perte de qualité de surface des cheveux est visible et inesthétique. Les cheveux reflètent en effet également moins la lumière et sont ainsi visiblement moins brillants, moins lumineux. Ils sont également plus fins et moins épais.

On entend ici par « annexes cutanées » les cheveux, les cils, les sourcils, les poils, notamment poils de la barbe, et/ou les ongles. Selon l'invention, la barbe inclue la moustache Préférentiellement, il s'agit des cheveux. L'hydrolysat selon l'invention est un ingrédient topiquement acceptable. On entend par « topiquement acceptable », un ingrédient adapté à une application par voie topique, non toxique, non irritant pour la peau, en particulier le cuir chevelu, ou les muqueuse ou les annexes cutanées, n'induisant pas de réponse allergique ou inflammatoire, qui n'est pas instable sur le plan chimique.

L'utilisation de l'hydrolysat peut être par voie orale ou topique. Avantageusement, elle est par voie topique. On entend par « voie topique », l'application locale directe et/ou la vaporisation de l'ingrédient sur la surface de la peau et/ou des muqueuses et/ou des annexes cutanées, en particulier des annexes cutanées. Selon un mode très avantageux, la voie topique exclue l'application sur la peau, incluant le cuir chevelu et/ou les muqueuses.

L'hydrolysat peut être appliqué par voie topique sur tout ou partie de la peau du corps et/ou du visage choisie parmi le cuir chevelu, les jambes, les cuisses, les bras, le ventre, le décolleté, le cou, tout ou partie du visage, le front, le menton, les lèvres, le contour des lèvres, le contour des yeux, la zone dite « en T » du visage, et avantageusement le cuir chevelu, et/ou sur tout ou partie des annexes cutanées, avantageusement sur les ongles, les cheveux, les poils, notamment les poils de barbe, des cils et/ou des sourcils, encore plus avantageusement sur les cheveux, plus préférentiellement les cheveux.

Par « peau » on entend donc aussi le cuir chevelu.

Un objet de l'invention concerne ainsi l'utilisation cosmétique non thérapeutique d'un hydrolysat de tourteau d'*H*. *rhamnoides* pour augmenter la croissance des annexes cutanées et/ou diminuer leur chute, avantageusement des cheveux; et/ou pour maintenir et/ou augmenter les propriétés biomécaniques et/ou les propriétés de surface et/ou de texture des annexes cutanées, avantageusement des cheveux ; et/ou pour réparer les annexes cutanées abîmées, avantageusement les cheveux.

On entend au sens de l'invention par « maintenir et/ou augmenter les propriétés biomécaniques » des annexes cutanées, maintenir et/ou augmenter leur résistance et/ou leur élasticité et/ou leur capacité de déformation, et/ou leur force, et/ou leur propriété de plasticité c'est-à-dire leur aspect cassant, en particulier leur réponse à l'étirement, et/ou leur souplesse. Ces propriétés peuvent être évaluées *ex-vivo* par la mesure de leur résistance aux forces de tension. Ces paramètres biomécaniques, notamment ceux des cheveux, peuvent être évalués en réponse à l'étirement et peuvent être mesurés par exemple par le test de traction (Dia-Stron). Les paramètres mesurés peuvent être le module élastique (Pa), l'allongement à la rupture (%), la force à rupture (gmf), le gradient de la région plastique post-yield (gmf / % allongement) et sont normalisés par rapport au diamètre des annexes cutanées. Ce dernier paramètre (gradient) permet la mesure de la plasticité du matériel d'étude juste avant la casse. La technique permet d'obtenir une courbe de contrainte (ou force) en fonction de l'allongement caractéristique du matériel étudié. Il est connu qu'une augmentation de l'allongement à la rupture et qu'une diminution du gradient post-yield peut s'observer sur des fibres capillaires (annexes cutanées) endommagées par oxydation, réduction ou irradiation ultraviolette suite à la casse des ponts disulfures en groupement libres dans le domaine cortical.

Dans un mode de réalisation de l'invention, « maintenir et/ou augmenter les propriétés biomécaniques » des annexes cutanées signifie diminuer les produits de glycation (Advanced Glycation End Products ou AGEs) qui apparaissent au cours du vieillissement chrono-induit ou photo-induit notamment, mais aussi qui apparaissent au quotidien en réponse aux conditions environnementales, au contact d'agents chimiques ou physiques oxydatifs. Ces produits de glycation diminuent les propriétés biomécaniques des annexes. Ainsi, dans un mode de réalisation, l'hydrolysat selon l'invention est en quantité efficace pour « maintenir et/ou augmenter les propriétés biomécaniques des annexes cutanées » lorsque le pourcentage de produits de glycation mesuré suite à l'oxydation de l'albumine comme protéine marqueur, en présence de fer comme catalyseur et de peroxyde d'hydrogène comme agent oxydant, et en présence de l'hydrolysat selon l'invention, est diminué d'au moins 20%, avantageusement d'au moins 25% et encore avantageusement d'au moins 50%, par rapport au pourcentage de produits de glycation détectés dans les mêmes conditions mais sans extrait. Avantageusement, les annexes cutanées sont les cheveux. Encore avantageusement, il s'agit d'une diminution des produits de glycation détectés en présence de l'hydrolysat tel que préparé selon l'exemple 1a) et dans les conditions décrites dans l'exemple 3a) (Tableau 3).

Dans un mode alternatif de réalisation de l'invention, « maintenir et/ou augmenter les propriétés biomécaniques » des annexes cutanées signifie diminuer la dégradation du tryptophane mesurée en présence de l'hydrolysat selon l'invention suite à l'oxydation de l'albumine en présence de peroxyde d'hydrogène et de fer comme catalyseur d'oxydation, d'au moins 30%, avantageusement d'au moins 40% et encore avantageusement d'au moins 95%, par rapport au pourcentage de dégradation du tryptophane mesuré en l'absence de l'hydrolysat selon l'invention. Avantageusement, les annexes cutanées sont les cheveux. Encore avantageusement, il s'agit de l'hydrolysat tel que préparé selon l'exemple 1a), dans les conditions décrites dans l'exemple 3b) (Tableau 4).

Dans un autre mode alternatif de réalisation de l'invention, « maintenir et/ou augmenter les propriétés biomécaniques des annexes cutanées » signifie diminuer la formation de dityrosine détectée en présence de fer et de peroxyde d'hydrogène après oxydation de l'albumine, d'au moins 35%, préférentiellement d'au moins 55%, en présence de l'hydrolysat selon l'invention. Dans un mode de réalisation avantageux, les annexes cutanées sont les cheveux. Encore avantageusement, l'hydrolysat est l'hydrolysat tel que préparé selon l'exemple 1a), dans les conditions décrites dans l'exemple 3c) (Tableau 5).

Dans encore un autre mode alternatif de réalisation de l'invention, « maintenir et/ou augmenter les propriétés biomécaniques des annexes cutanées » signifie diminuer la formation de pentosidine détectée en présence de fer après oxydation de l'albumine en présence de peroxyde d'hydrogène, d'au moins 35%, préférentiellement d'au moins 60%, en présence de l'hydrolysat selon l'invention. Dans un mode de réalisation avantageux, les annexes cutanées sont les cheveux. Encore avantageusement, l'hydrolysat est l'hydrolysat tel que préparé selon l'exemple 1a), dans les conditions décrites dans l'exemple 3d) (Tableau 6).

Dans encore un autre mode de réalisation de l'invention, « maintenir et/ou augmenter les propriétés biomécaniques des annexes cutanées » signifie diminuer la formation des bases de Schiff, produits intermédiaires de glycation, en présence de cuivre et de lipoprotéines, d'au moins 11%, avantageusement d'au moins 18%, très avantageusement d'au moins 93% en présence de l'hydrolysat selon l'invention, versus la quantité desdites bases de Schiff mesurée dans les mêmes conditions sans hydrolysat. Dans un mode de réalisation avantageux, les annexes cutanées sont les cheveux. Encore avantageusement, l'hydrolysat est l'hydrolysat tel que préparé selon l'exemple 1a), dans les conditions décrites dans l'exemple 3d) (Tableau 7).

L'hydrolysat selon l'invention est donc efficace et peut être utilisé pour augmenter la résistance, l'élasticité, la souplesse, la force et/ou la plasticité des annexes cutanées, et préférentiellement celles des cheveux, et est ainsi capable de diminuer leur casse.

On entend encore par « maintenir et/ou augmenter les propriétés de surface et/ou de texture » des annexes cutanées, avantageusement les cheveux, rendre lesdites annexes cutanées plus lisses et/ou plus douces et/ou moins rugueuses et donc visuellement plus brillantes et/ou plus lumineuses et ainsi faciles à démêler et/ou faciles à coiffer et/ou faciles à mettre en forme. Ces propriétés peuvent être évaluées par technique d'imagerie, par exemple par vidéo-microscopie ou microscopie électronique, ou par des tests sensoriels sur mèches de cheveux, réalisés par des volontaires externes entrainés à définir leurs sensations visuelles ou tactiles ou encore par des tests d'évaluation par des experts (coiffeurs) ou d'auto-évaluation par questionnaires consommateurs. L'efficacité d'un produit dans sa formule est évaluée selon des critères de qualité visuelle ou tactiles perceptibles de la chevelure.

On entend en outre par « maintenir et/ou augmenter la croissance des annexes cutanées » maintenir et/ou augmenter la quantité d'ADN au niveau des fibroblastes de la papille des follicules pileux. Dans un mode de réalisation, il s'agit d'une augmentation de la quantité d'ADN d'au moins 20%, avantageusement d'au moins 30% et encore avantageusement d'au moins 40%, mesurée dans les fibroblastes de la papille de cheveu en présence de l'hydrolysat selon l'invention en comparaison de la quantité d'ADN mesurée en l'absence de l'hydrolysat sur des fibroblastes non sensibilisés. Encore avantageusement, l'hydrolysat selon l'invention est celui décrit dans l'exemple 1a), dans les conditions de mesure telles qu'exposées dans l'exemple 4 (Tableau 8).

Dans un mode alternatif de réalisation de l'invention, « maintenir et/ou augmenter la croissance des annexes cutanées » signifie augmenter la quantité d'ATP dans les fibroblastes de la papille des follicules pileux. Dans un mode de réalisation, il s'agit d'une augmentation de la quantité d'ATP mesurée au niveau des fibroblastes de la papille de cheveux en présence de l'hydrolysat selon l'invention, d'au moins 10%, préférentiellement d'au moins 15% et encore préférentiellement d'au moins 40%, en comparaison de la quantité d'ATP mesurée sans hydrolysat. Encore avantageusement, l'hydrolysat selon l'invention est celui décrit dans l'exemple 1a), dans les conditions de mesure telles qu'exposées dans l'exemple 4 (Tableau 9).

Dans encore un autre mode alternatif de réalisation de l'invention, « maintenir et/ou augmenter la croissance des annexes cutanées » signifie maintenir et/ou augmenter la synthèse des fibroblastes au niveau de la papille des follicules pileux desdites annexes. Dans un mode de réalisation de l'invention, il s'agit d'une augmentation du nombre de fibroblastes de la papille d'au moins 10%, préférentiellement d'au moins 20%, encore préférentiellement d'au moins 25%, en présence de l'hydrolysat selon l'invention, en comparaison du nombre de fibroblastes mesuré sans hydrolysat. Avantageusement, il s'agit d'une augmentation du nombre de fibroblastes mesurée au niveau de la papille des follicules du cheveu. Encore avantageusement, cette augmentation est mesurée en présence de l'hydrolysat préparé selon l'exemple 1a), dans les conditions décrites dans l'exemple 4 (Tableau 10).

L'effet de l'hydrolysat sur l'augmentation de la croissance des annexes cutanées, en particulier des cheveux, peut aussi être évalué *ex-vivo.* Ainsi dans encore un autre mode alternatif de réalisation de l'invention, on entend par « augmenter la croissance des annexes cutanées », une élongation des fibres kératiniques d'au moins 5%, avantageusement d'au moins 10% en présence de l'hydrolysat selon l'invention, avantageusement en présence d'un agent sensibilisant lesdites fibres et ralentissant leur élongation, en comparaison de l'élongation de fibres kératiniques contrôle sensibilisées avec ledit agent mais sans hydrolysat. Avantageusement, il s'agit de cheveux. Très avantageusement, l'agent sensibilisant est la capsaïcine. Encore avantageusement, l'hydrolysat de tourteau d'*H*. *rhamnoides* est celui préparé tel que décrit dans l'exemple 1a), dans les conditions exposées dans l'exemple 5.

On entend en outre par « diminuer la chute des annexes cutanées » augmenter la quantité d'ADN au niveau des fibroblastes de la papille des follicules pileux, lorsqu'ils sont sensibilisés avec un agent dédié. Avantageusement ainsi, il s'agit d'une augmentation d'au moins 25%, avantageusement d'au moins 50% et très avantageusement d'au moins 60%, de la quantité d'ADN mesurée au niveau de fibroblastes sensibilisés par la capsaïcine et en présence de l'hydrolysat selon l'invention, en comparaison de la quantité d'ADN mesurée au niveau des mêmes fibroblastes sensibilisés par la capsaïcine et sans hydrolysat. Dans un mode de réalisation particulièrement avantageux de l'invention, il s'agit d'une augmentation de la quantité d'ADN mesurée au niveau de la papille des follicules du cheveu. Encore avantageusement, cette augmentation est mesurée en présence de l'hydrolysat préparé selon l'exemple 1a), dans les conditions décrites dans l'exemple 4 (Tableau 8).

Dans un mode alternatif de l'invention, « diminuer la chute des annexes cutanées » signifie augmenter le nombre de fibroblastes de la papille sensibilisés par la capsaïcine d'au moins 20%, préférentiellement d'au moins 30%, encore préférentiellement d'au moins 50%, en présence de capsaïcine et de l'hydrolysat selon l'invention, en comparaison du nombre de fibroblastes sensibilisés par la capsaïcine et sans hydrolysat. Dans un mode de réalisation particulièrement avantageux de l'invention, il s'agit d'une augmentation du nombre de fibroblastes mesurée au niveau de la papille des follicules du cheveu. Encore avantageusement, cette augmentation est mesurée en présence de l'hydrolysat préparé selon l'exemple 1a), dans les conditions décrites dans l'exemple 4 (Tableau 10). Dans encore un autre mode alternatif de réalisation de l'invention, « diminuer la chute des annexes cutanées » signifie augmenter la quantité d'ATP dans les fibroblastes de la papille des follicules pileux, préférentiellement lorsqu'ils sont sensibilisés à la capsaïcine. Dans un mode de réalisation avantageux, il s'agit d'une augmentation de la quantité d'ATP mesurée au niveau des fibroblastes de la papille de cheveu, avantageusement soumis à une sensibilisation à la capsaïcine, en présence de l'hydrolysat selon l'invention, d'au moins 100%, préférentiellement d'au moins 130% mesurée au niveau de fibroblastes sensibilisés par la capsaïcine et en présence de l'hydrolysat selon l'invention, en comparaison de la quantité d'ATP mesurée au niveau des mêmes fibroblastes sensibilisés par la capsaïcine sans hydrolysat. Dans un mode de réalisation particulièrement avantageux de l'invention, il s'agit d'une augmentation de la quantité d'ATP mesurée au niveau de la papille des follicules du cheveu. Encore avantageusement, cette augmentation est mesurée en présence de l'hydrolysat préparé selon l'exemple 1a), dans les conditions décrites dans l'exemple 4 (Tableau 9).

Les propriétés d'augmentation de la croissance des annexes cutanées, en particulier des cheveux, ainsi que d'augmentation des propriétés biomécaniques et/ou de surface et/ou de texture de l'hydrolysat selon l'invention en font un ingrédient actif sur l'amélioration du volume, de la couleur, de la brillance et de l'éclat des annexes cutanées, et en particulier des cheveux.

L'efficacité de l'hydrolysat selon l'invention sur les propriétés biomécaniques des annexes cutanées, en particulier des cheveux, peut être par ailleurs mise en évidence par différents tests dont des tests de force de tension (Diastron), des tests de fatigue, ou des tests de coiffage répétés des cheveux permettant d'évaluer leur casse. Il est possible aussi de quantifier les fibres capillaires comportant des fourches. On pourra par ailleurs évaluer ces propriétés biomécaniques en mesurant la force de coiffage nécessaire sur cheveux secs ou mouillés.

En outre, il est possible d'évaluer la pénétration de l'hydrolysat en mesurant directement sa distribution au niveau des annexes cutanées. Avantageusement, la distribution de l'hydrolysat est étudiée au niveau des cheveux par différentes techniques d'imagerie et de microscopie. Encore avantageusement, la spectrométrie infrarouge à transformée de Fourier (IRTF) peut être utilisée. Ainsi dans un mode de réalisation avantageux de l'invention, les cheveux sont étudiés par IRTF après avoir été coupé transversalement, après application de l'hydrolysat selon l'invention sur des cheveux endommagés par un agent oxydant, en comparaison des mêmes cheveux endommagés par le même agent oxydant sans application de l'hydrolysat, ou de cheveux non endommagés. Les profils vibrationnels de l'hydrolysat sont ensuite comparés aux profils vibrationnels de la coupe pour évaluer la distribution du produit dans les cheveux. Avantageusement encore, l'agent oxydant est le peroxyde d'hydrogène.

On entend en outre par « réparer les annexes cutanées abîmées » un effet réparateur de l'hydrolysat selon l'invention sur des annexes cutanées endommagées par les carences nutritionnelles, les polluants choisis parmi les particules fines dites PM2,5 et PM10, les métaux, tels que par exemple le cuivre ou le fer, les métaux lourds, les conditions climatiques et/ou environnementales telles que le vent, la pluie, les variations brutales de températures et d'humidité, le froid, la chaleur, les UV, le sel marin, le chlore des piscines et d'autres agents physiques ou les agents chimiques, dont le peroxyde d'hydrogène en particulier présent dans certains shampoings, les produits cosmétiques de maquillage, les produits de coloration des cheveux, de décoloration, de permanente, les vernis, dissolvants et produits ménagers, les agressions mécaniques telles que les frottements avec le brossage, la chaleur avec les sèche-cheveux et fer à lisser par exemple mais aussi le vieillissement intrinsèque et/ou chrono-induit. Ce dernier est en effet aussi responsable de la perte des qualités structurelles et/ou fonctionnelles des annexes cutanées, dont la perte d'hydratation, en particulier des cils, sourcils et cheveux qui se détériorent. Au sens de la présente invention, on entend par « réparer les annexes cutanées » améliorer au moins partiellement la structure et/ou les propriétés visuelles et/ou les propriétés biomécaniques des annexes cutanées, préférentiellement des cheveux. Cette réparation est préférentiellement évaluée lorsque les annexes cutanées sont exposés à des agressions environnementales, chimiques biologiques et/ou mécaniques.

Les agressions environnementales incluent notamment les fumées, la pollution, la température notamment le chaud et le froid et leurs variations brutales, la teneur en eau notamment l'humidité ou la sècheresse, les irradiations solaires notamment les spectres visibles, les UV et/ou les rayons gamma, la pluie, le vent, la poussière, et le sel marin.

Les agressions chimiques incluent les produits ménagers agressifs, le chlore des piscines, les produits cosmétiques agressifs tels que les colorations et décolorations, les vernis et/ou dissolvants, les solvants, les produits de maquillage.

Les agressions chimiques des cheveux incluent en particulier les shampoings et soins et/ou traitement capillaires agressifs notamment pour le coiffage, la mise en forme telle que le lissage et/ou les permanentes et/ou pour la coloration et/ou la décoloration.

Les agressions mécaniques incluent notamment le frottement tel que le brossage et/ou le frottement sur les tissus (oreilles, vêtements) et/ou des particules comme la poussière et/ou le sable, la chaleur avec les sèche-cheveux et fer à lisser, et/ou le coiffage avec notamment l'exposition à des forces de traction, étirement et/ou torsions.

L'hydrolysat selon l'invention est aussi efficace en tant qu'ingrédient anti-âge des annexes cutanées, avantageusement des cheveux, et/ou de la peau, avantageusement du cuir chevelu, et/ou des muqueuses, en diminuant les effets négatifs du vieillissement.

Ainsi dans un mode de réalisation de l'invention, l'effet réparateur peut être évalué selon les techniques existantes dans le domaine. Les méthodes classiques de mesure d'un effet de réparation permettent de mesurer la capacité du produit évalué à restaurer un état visuel, structurel et/ou fonctionnel d'une annexe cutanée endommagée, préférentiellement de cheveux endommagés comparable à l'état d'une annexe cutanée non endommagée, préférentiellement de cheveux non endommagés. Elles sont mises en œuvre sur annexes cutanées, préférentiellement cheveux, endommagé(e)s et l'effet de réparation est mesuré par comparaison aux annexes cutanées, préférentiellement cheveux, non endommagé(e)s. Avantageusement l'effet réparateur est un effet réparateur des annexes cutanées endommagées *in vitro* par un agent oxydant, lequel induit une dénaturation des protéines des annexes. Avantageusement, il s'agit d'un effet réparateur de l'hydrolysat sur les cheveux et préférentiellement encore l'agent oxydant est le peroxyde d'hydrogène. Encore avantageusement, cette dénaturation protéique est évaluée par la mesure de la température de dénaturation (°C) protéique par calorimétrie différentielle (Wortmann et Deutz, Appl. Polym. Sci., 48, 137-150 (1993)) à l'aide d'un analyseur enthalpique différentiel (DSC Q100, TA Instruments), dans les conditions décrites dans l'exemple 6. Ainsi l'hydrolysat selon l'invention est en quantité efficace pour réparer les annexes cutanées, préférentiellement les cheveux, lorsque l'indice de réparation des annexes endommagées est d'au moins 20%, préférentiellement d'au moins 30% par rapport à des annexes dites saines, c'est-à-dire non soumises au stress oxydatif induit par l'eau oxygénée. Avantageusement, il s'agit de la mesure de l'indice de réparation en présence de l'hydrolysat tel que préparé selon l'exemple 1a), dans les conditions décrites dans l'exemple 6.

Pour évaluer cet effet de réparation des annexes cutanées *in vivo,* avantageusement des cheveux, plusieurs méthodes peuvent être utilisées choisies parmi la vidéo microscopie, la microscopie confocale, la microscopie IRTF ou Raman, les rayons X, et la microscopie électronique, qui a pour but notamment d'observer l'état et/ou la qualité de la cuticule, enveloppe protectrice de la fibre kératinique des annexes cutanées, par visualisation et quantification des écailles décollées comme témoin de l'endommagement de surface. Des méthodes de quantification physique de surface permettant d'évaluer la morphologie des annexes cutanées, en particulier des cheveux, telles que l'Atomic Force Microscopy ou la White-light interferometric profilometry, d'évaluer la chimie (XPS), la charge (Streaming potential) ou encore l'énergie (inverse Gas Chromatography) peuvent être mises en place.

Pour évaluer l'effet de réparation de l'hydrolysat selon l'invention sur les propriétés internes des annexes cutanées, en particulier des cheveux, la calorimétrie (DSC) peut être utilisée.

L'hydrolysat selon l'invention est un hydrolysat obtenu par hydrolyse enzymatique ou chimique du coproduit d'extraction au CO₂ supercritique des graines d'*H*. *rhamnoides,* c'est à dire du coproduit de l'huile, ou du coproduit obtenu après pressage desdites graines. Au sens de la présente invention, la graine correspond à la baie (ou fruit) d'*H*. *rhamnoides* débarrassée de la partie charnue, autrement dénommée péricarpe. L'extraction supercritique au dioxyde de carbone est une technique connue de l'homme du métier permettant d'isoler la fraction huileuse du composé à extraire pour l'utiliser pour diverses applications. Le résidu de cette extraction est appelé « coproduit » au sens de la présente invention et contient l'ensemble des composés non extraits par ladite technique. Par coproduit, on entend donc le résidu obtenu après extraction de l'huile, aussi appelé tourteau. Le terme de tourteau sera utilisé dans la suite de la description. Avantageusement il s'agit donc de la fraction délipidée de la graine d'*H*. *rhamnoides.* Préférentiellement, il s'agit de l'hydrolysat enzymatique ou chimique du coproduit d'extraction au CO₂ supercritique des graines d'*H*. *rhamnoides,* encore préférentiellement de son hydrolysat enzymatique. Plus préférentiellement, le co-produit ne contient pas d'huile. L'hydrolyse enzymatique peut être conduite en présence de toute protéase connue de l'Homme du métier, et avantageusement en présence d'une enzyme d'origine animale choisie parmi la pepsine, les enzymes d'origine pancréatique telles que la trypsine ou la chymotrypsine, et préférentiellement la trypsine, d'origine végétale choisie parmi la papaïne, la bromélaïne, la ficine, l'actinidine, préférentiellement la papaïne, ou encore d'origine bactérienne choisie parmi l'enzyme provenant de la souche *Bacillus licheniformis* commercialisée sous le nom d'Alcalase^{®} ou de la souche *B. subtilis,* préférentiellement l'Alcalase^{®}. Dans un mode de réalisation particulièrement avantageux de réalisation de l'invention, l'enzyme utilisée est l'Alcalase^{®}.

L'hydrolyse peut être conduite à un pH compris entre 3 et 9 selon le pH optimum de l'enzyme, et avantageusement à un pH compris entre 4 et 8,5, très avantageusement à un pH de 8,5. L'hydrolyse peut être réalisée à une température de 40°C à 65°C, avantageusement de50 à 60°C et très avantageusement à 55°C. Elle est conduite durant ure période de 1 heure à 3 heures et préférentiellement durant une période de 2 heures.

L'enzyme utilisée est ensuite inactivée par chauffage, avantageusement à une température de 80°C à 100°C et très avantageusement à une température de 90°C, durant une période de 5 minutes à 30 minutes, préférentiellement durant une période de 10 minutes. L'inactivation de l'enzyme a lieu à pH compris entre 5 et 8, préférentiellement à pH 6,5.

L'hydrolysat obtenu est ensuite centrifugé puis purifié par filtrations successives jusqu'à une porosité de 0,22µm.

Avantageusement, avant l'étape d'hydrolyse enzymatique, les protéines sont préalablement extraites du tourteau. Dans ce cas, elles sont extraites à un pH compris entre 7,5 et 10, avantageusement à un pH compris entre 8 et 9, très avantageusement à un pH de 9,0, durant une période de 30 minutes à 2 heures, préférentiellement durant une période de 1 heure.

La quantité en poids de tourteau utilisée pour l'extraction des protéines et leur hydrolyse est comprise entre 5% et 20%, avantageusement comprise entre 5% et 15%, encore avantageusement elle est de 10%, en poids par rapport au poids total du solvant et du tourteau.

Le solvant utilisé pour l'extraction protéique est choisi parmi l'eau, l'eau de coco en tant que solvant tel que décrit dans la demande FR3061416, ou encore un solvant comprenant au moins 50 % en poids par rapport au poids total du solvant d'au moins un carbonate de dialkyle en C₆-C₁₆ tel que décrit dans la demande FR3069450, parmi lesquels un carbonate de dialkyle en C₇-C₁₀, avantageusement en C₈, par exemple le carbonate de dioctyle ou le carbonate de diéthylhexyle. Avantageusement, le solvant utilisé est l'eau comme unique solvant.

Ladite extraction protéique peut être conduite à une température de 4°C à 300°C, avantageusement de 4°C à 100°C, encore avantageusement de 15°C à 80°C, très avantageusement de 15°C à 30°C, yinclus la température ambiante, c'est-à-dire à une température de 20°C. Dans un mode de réalisation particulièrement avantageux de l'invention, l'extraction protéique est conduite à température ambiante.

Dans un mode de réalisation alternatif, l'extraction est effectuée en conditions subcritiques.

On entend par extraction en « conditions subcritiques » une extraction en présence d'eau, dans des conditions de température supérieures à 100°C et de pression inférieure à 22,1 MPa (221 bars), telle que l'eau reste à l'état liquide mais possède une viscosité et une tension de surface inférieures à celle de l'eau à température ambiante, augmentant sa constante diélectrique. Ainsi, la pression d'extraction sera comprise entre 10 MPa (100 bars) et 25 MPa (250 bars), préférentiellement entre 15 et 22,1 MPa (150 et 221bars).

Ainsi en conditions subcritiques, l'extraction est réalisée dans l'eau à une température allant de 100°C à 300°C, avantageusement de 120°C à 250°C, encore avantageusement entre 140°C et 200°CL'extraction peut être réalisée à une température donnée unique ou à des températures successives croissantes. Dans un mode de réalisation avantageux de l'invention, l'extraction sera réalisée à une température unique de 160°C. Dans un mode alternatif, elle sera conduite selon un gradient de trois températures croissantes comprises entre 100°C et 200°C, tel que 120°C, 140°C puis 160°C ou 110°C, 130°C puis 150°C, ou eoœ 120°C, 145°C puis 170°C.

Dans un mode particulièrement avantageux de réalisation de l'invention, l'hydrolysat est obtenu par digestion enzymatique comme suit : les protéines sont extraites durant une période d'une heure à température ambiante, à pH 12, à partir d'une quantité de 10% en poids de tourteau par rapport au poids total de tourteau et d'eau comme solvant. Les protéines ainsi extraites sont soumises à hydrolyse durant une période de 2 heures à une température de 55°C et à pH 8,5, en présence d'une concentration en volume de 5% d'Alcalase^{®} par rapport au volume total d'Alcalase^{®} et de protéines. L'enzyme est inactivée par chauffage durant une période de 20 minutes à une température de 90°C et à pH 6,5. Le mélange est ensuite refroidi, centrifugé, filtré (0,22µm), dans les conditions telles que décrites dans l'exemple 1a).

Alternativement, les protéines du tourteau sont extraites durant une période d'une heure à température ambiante, c'est-à-dire à une température de 20°C, à pH 8,5, à partir d'une quantté de 10% en poids de tourteau par rapport au poids total de tourteau et d'eau comme solvant. Les protéines ainsi extraites sont soumises à hydrolyse durant une période de 2 heures à une température de 55°C et à pH 8,5, en présence d'une concentration en volume de 5% d'Alcalase^{®} par rapport au volume total d'Alcalase^{®} et de protéines. L'enzyme est inactivée par chauffage durant une période de 20 minutes à une température de 90°C et à pH 6,5. Le mélange est ensuite refroidi, centrifugé, filtré (0,22µm) dans les conditions décrites dans l'exemple 1b).

Alternativement encore, les protéines du tourteau sont extraites durant une période d'une heure à température ambiante, c'est-à-dire à une température de 20°C, à pH 12, à partir d'une quantté de 10% en poids de tourteau par rapport au poids total de tourteau et d'eau comme solvant. Les protéines ainsi extraites sont soumises à hydrolyse durant une période de 2 heures à une température de 55°C et àpH 8,5, en présence d'une concentration en volume de 5% de papaïne par rapport au volume total de papaïne et de protéines. La papaïne est inactivée par chauffage durant une période de 20 minutes à une température de 90°C et à pH 6,5. Le mélange est ensuite refroidi, centrifugé, filtré (0,22µm) et dans les conditions telles que décrites dans l'exemple 1c).

L'hydrolysat selon l'invention comprend par conséquent une teneur en matière sèche de 1% à 20% en poids, avantageusement de 2 à 10% en poids, y inclus 5%, une teneur en protéines totales de 15 g/L à 35 g/L d'hydrolysat, avantageusement de 18,8 g/L d'hydrolysat, ainsi qu'un pourcentage en peptides de poids moléculaire de 5kDalton (Da) à 30kDa compris entre 15 et 40%, avantageusement de 18,25%, un pourcentage en peptides de poids moléculaire de moins de 5KDa compris entre 40% et 85%, avantageusement de 72,6% (Exemple 2). Avantageusement, l'hydrolysat comprend une teneur en matière sèche d'au moins 3% en poids, en particulier d'au moins 3,5% en poids, plus particulièrement de 3,44% en poids, très avantageusement de 3,84% en poids. Dans un mode encore plus avantageux, l'hydrolysat comprend une teneur en matière sèche de 5% en poids.

Très avantageusement, l'hydrolysat comprend un pourcentage en peptides de poids moléculaire de 5kDa à 30kDa de 18,25%, et un pourcentage en peptides de poids moléculaire de moins de 5KDa de 72,6%.

Encore très avantageusement, l'hydrolysat selon l'invention ne contient pas d'acide gallique, ni l'un quelconque des polymère hydrolysable en acide gallique, ni d'acide hexahydroxydiphénique ni l'un quelconque des polymères hydrolysables en acide hexahydroxydiphénique, ni d'acide ellagique ni l'un quelconque des polymères hydrolysables en acide ellagique, ni de gallotanins, ni d'ellagitanins. Encore avantageusement l'hydrolysat ne contient pas non plus l'un quelconque des dérivés des composés cités ni l'un quelconque de leurs sels, en particulier tels que décrit dans la demande de brevet FR031455.

Dans un autre mode de réalisation avantageux, l'hydrolysat selon l'invention ne contient pas de L-quebrachitol, ni d'autres methyl inositols et/ou inositols tels que décrits dans WO2009/125071. En particulier l'hydrolysat selon l'invention ne contient pas de myricétine, ni de quercetine, ni de Kampfaerol ni d'isorhamnetine.

L'hydrolysat, en particulier tel que préparé dans les exemples 1a) à 1c), se présente ainsi sous forme liquide. Optionnellement, l'hydrolysat peut être ensuite séché par exemple par lyophilisation ou par atomisation en présence de maltodextrines. L'hydrolysat se présente alors sous forme de poudre.

Dans ce cas, l'hydrolysat selon l'invention, en particulier obtenu dans les conditions décrites dans les exemples 1a) à 1c), est atomisé en présence d'une concentration en poids de maltodextrines comprise entre 20% et 90%, préférentiellement entre 40 et 80%, encore préférentiellement de 70 à 80% par rapport au poids total de la poudre obtenue.

L'hydrolysat selon l'invention peut être utilisé seul ou incorporé dans une composition cosmétique.

Lorsqu'il est utilisé seul sous forme d'ingrédient cosmétique ou dermatologique, il est préférentiellement solubilisé dans une solution aqueuse contenant de la glycérine, avantageusement présente à une concentration de 60% à 90%, encore avantageusement de 70% à 85%, très avantageusement à une concentration de 80% en poids par rapport au poids total de la solution aqueuse comprenant l'hydrolysat.

Dans un mode de réalisation alternatif de l'invention, l'hydrolysat sera solubilisé et/ou dilué dans un solvant notamment polaire, tel que l'eau, un alcool, un polyol, un glycol, tel que le pentylène glycol et/ou le butylène glycol et/ou le propylène glycol et/ou l'hexylène glycol et/ou le caprylyl glycol, ou un de leurs mélanges, préférentiellement un mélange hydroglycolique, encore préférentiellement contenant un glycol choisi parmi l'hexylène glycol, le propylène glycol, le caprylyl glycol et l'un quelconque de leurs mélanges. Avantageusement l'hydrolysat obtenu est dilué et/ou soluble dans une solution aqueuse contenant de l'hexylène glycol, en particulier contenant entre 0,1 et 10 % en poids de l'hexylène glycol, préférentiellement entre 0,5 et 5 % en poids d'hexylène glycol, par rapport au poids total de l'ingrédient cosmétique. Avantageusement l'hydrolysat obtenu est dilué et/ou soluble dans une solution aqueuse contenant du caprylyl glycol, en particulier contenant entre 0,01 et 5 % en poids de caprylyl glycol, préférentiellement entre 0,1 et 1 % en poids de caprylyl glycol, par rapport au poids total de la solution aqueuse comprenant l'hydrolysat. Alternativement, la solution dans laquelle est solubilisé l'hydrolysat selon l'invention comprend du propylène glycol et du caprylyl glycol.

De façon particulière, la solution aqueuse dans laquelle est solubilisée l'hydrolysat selon l'invention comprend de la gomme xanthane, en particulier entre 0,01 et 5 % en poids de gomme xanthane par rapport au poids total la solution aqueuse, plus particulièrement entre 0,1 et 1 % en poids de gomme xanthane par rapport au poids total de la solution aqueuse comprenant l'hydrolysat.

De façon avantageuse, la solution dans laquelle est solubilisé l'hydrolysat selon l'invention comprend de l'hexylène glycol, du caprylyl glycol et de la gomme de xanthane.

Dans un mode alternatif de réalisation de l'invention, l'extrait est solubilisé dans une solution comprenant un mélange de benzoate de sodium et de glucunolactone commercialisé sous le nom de Geogard^{™}.

L'hydrolysat peut être incorporé dans une composition cosmétique comprenant au moins un excipient cosmétiquement acceptable. On entend au sens de la présente invention par excipient « cosmétiquement acceptable » un composé et/ou solvant topiquement acceptable, c'est-à-dire n'induisant pas de réponse inflammatoire et allergique au contact de la peau, notamment du cuir chevelu, non toxique, non instable, ou leurs équivalents, indue.

On entend au sens de la présente invention par composition cosmétique une composition non thérapeutique, c'est-à-dire une composition destinée à la prévention et/ou au soin de la peau, notamment du cuir chevelu, et/ou des annexes cutanées dite « normale » par un dermatologue, c'est-à-dire non pathologique. On entend ici par peau ou cuir chevelu ou annexe cutanée « normal(e) » une peau ou un cuir chevelu ou une annexe cutanée sain(e) tel que défini précédemment.

Dans un mode de réalisation préférentiel de l'invention, l'hydrolysat selon l'invention est présent dans la composition cosmétique en une teneur comprise entre 1×10⁻⁴% à 10% en poids, préférentiellement de 1×10⁻⁴% à 5% en poids, encore avantageusement de 1×10⁻³% à 3% en poids, encore préférentiellement de 0,001% et 0,1 % en poids, par rapport au poids total de la composition.

La composition peut donc être utilisée pour augmenter la croissance des annexes cutanées et/ou diminuer leur chute, avantageusement des cheveux ; et/ou pour maintenir et/ou augmenter les propriétés biomécaniques et/ou les propriétés de surface et/ou de texture des annexes cutanées, avantageusement des cheveux ; et/ou pour réparer les annexes cutanées abîmées, avantageusement les cheveux.

La composition cosmétique selon l'invention, peut se présenter sous les formes galéniques classiquement utilisées pour une application topique sur la peau ou le cuir chevelu et/ou les annexes cutanées, préférentiellement le cuir chevelu et/ou les annexes cutanées, telles que les formes liquides ou solides ou même sous la forme de liquide sous pression. Elles peuvent être formulées sous la forme d'une solution, aqueuse ou huileuse, une crème ou un gel aqueux ou un gel huileux, notamment en pot ou en tube, notamment un gel douche, un shampoing, un après-shampoing, un lait, une huile, une émulsion, un hydrogel, une microémulsion ou une nano-émulsion, notamment huile-dans-eau ou eau-dans-huile ou multiple ou siliconée, un sérum, une lotion, notamment en flacon de verre, de plastique ou en flacon doseur ou en aérosol ou en spray, une ampoule, un savon liquide, une pâte, un pain dermatologique, une pommade, une mousse, un masque, une laque, un patch, un vernis, un produit anhydre, de préférence liquide, pâteux ou solide, par exemple sous forme de bâtonnet notamment en stick ou en poudres. Il peut s'agir d'un produit de maquillage en particulier pour cils ou sourcils tels qu'un mascara ou un crayon ou d'un produit de démaquillage. En particulier, la composition cosmétique est choisie dans le groupe constitué par un sérum, une lotion, une crème, un shampoing, un après-shampoing, une huile, un lait, une pommade, une pâte, une mousse, une émulsion, un hydrogel, un gel douche, un masque, une laque, un spray, une cire, un mascara, un crayon de maquillage, un vernis, avantageusement sous forme d'un shampoing, après-shampoing ou d'une lotion.

De manière préférentielle l'hydrolysat est adapté pour la formulation de composition dite neutre et douce pour le respect de la fibre kératinique, notamment de la fibre capillaire, et de la peau, notamment du cuir chevelu. L'hydrolysat est également adapté pour l'utilisation dans des formulations cationiques avec des tensioactifs.

Les compositions selon l'invention peuvent contenir tout solvant approprié et/ou tout véhicule approprié et/ou tout excipient approprié, éventuellement en combinaison avec d'autres composés d'intérêts. Elles peuvent notamment contenir un excipient cosmétiquement acceptable choisis parmi des agents tensioactifs, des conservateurs, des agents tampon, des agents gonflants, des agents chélatants, des agents biocides, des dénaturants, des agents opacifiants, des ajusteurs de pH, des agents réducteurs, des agents stabilisants, des émulsifiants, des épaississants, des gélifiants, des polymères filmogènes, des solvants, des charges, des bactéricides, des absorbeurs d'odeurs, des agents matifiants, des agents conditionneurs, des agents de texture, des agents de brillance, des pigments, des colorants, des parfums et des filtres solaires chimiques ou minéraux, des oligo-éléments, des huiles essentielles. Ces combinaisons sont également couvertes par la présente invention. Le CTFA Cosmetic Ingredient Handbook, Second Edition (1992) décrit différents ingrédients cosmétiques utilisés couramment dans l'industrie cosmétique, qui sont en particulier adaptés à une utilisation topique sur le cuir chevelu.

La composition cosmétique peut contenir d'autres agents cosmétiques possédant des propriétés identiques à l'hydrolysat selon l'invention et induisant un effet synergique ou non avec ledit hydrolysat, ou contenir des agents cosmétiques à effets complémentaires. A titre d'actif anti-chute, on citera l'association de sulfopeptides, acides aminés, aminosaccharides, vitamine du groupe B, zinc et extrait de *Panax ginseng* et *Artium majus* commercialisé sous le nom Trichogen^{™} LS 8960 par la demanderesse ou un agent protecteur capillaire tel qu'un extrait de péricarpe de *Litchi chinensis* commercialisé sous le nom Litchiderm^{™} par la demanderesse, un actif apaisant et anti-démangeaisons tels que les phytostérols de colza commercialisé sous le nom de Phytosoothe^{™} LS9766 par la demanderesse.

D'autres actifs pourront être présents dans la composition, tels qu'un extrait de feuilles de *Cassia alata* commercialisé sous le nom de DN-Age^{™} en tant qu'actif anti-oxydant pour le soin des cheveux notamment, une combinaison d'un extrait de *Salvia miltiorhizza* et de niacinamide commercialisée sous le nom de CollRepair^{™} en tant qu'agent déglyquant, ou des actifs favorisant la fermeté de la peau donc du cuir chevelu, tels qu'un tétrapeptide synthétique commercialisé sous le nom de Dermican^{™}, un extrait d'*Hibiscus abelmoschus* commercialisé sous le nom de Linefactor^{™}, un extrait purifié de pois commercialisé sous le nom de Proteasyl^{™}, un extrait de *Manilkara multinervis* commercialisé sous le nom d'Elestan^{™}, un extrait de *Khaya senegalensis* commercialisé sous le nom de Collalift^{™}18, un extrait de pulpe d'Argan commercialisé sous le nom d'Argassential^{™} par la demanderesse, un extrait de *Schizandra chinensis* commercialisé sous le nom de Sqisandryl^{™}, un extrait d*'Eperua falcata* commercialisé sous le nom d'Eperuline^{™}, ou encore un extrait d*'Orthosiphon staminus* commercialisé sous le nom de MAT-XS^{™} Bright commercialisés par la demanderesse. Ces associations d'actifs permettent notamment de renforcer le follicule pileux et diminuer la chute des cheveux. L'hydrolysat de l'invention peut aussi être combiné à un extrait de graines de la plante *Nephelium lappaceum,* commercialisé sous le nom de Rambuvital^{™} par la demanderesse pour ses propriétés protectrices des cheveux, contre la pollution notamment.

Un troisième objet de l'invention est relatif à un procédé de soin cosmétique, non thérapeutique, comprenant l'application par voie topique de l'hydrolysat selon l'invention ou d'une composition cosmétique le comprenant, pour augmenter la croissance des annexes cutanées et/ou diminuer leur chute, avantageusement des cheveux; pour maintenir et/ou augmenter les propriétés biomécaniques et/ou les propriétés de surface et/ou de texture des annexes cutanées, avantageusement des cheveux ; et/ou pour réparer les annexes cutanées abîmées, avantageusement les cheveux.

Dans un mode de réalisation avantageux de l'invention, le procédé de soin cosmétique consiste en l'application par voie topique de l'hydrolysat selon l'invention ou de la composition cosmétique le comprenant sur tout ou partie de la peau du corps et/ou du visage choisie parmi le cuir chevelu, les jambes, les cuisses, les bras, le ventre, le décolleté, le cou, tout ou partie du visage, le front, le menton, les lèvres, le contour des lèvres, le contour des yeux, la zone dite « en T » du visage, et avantageusement le cuir chevelu, et/ou sur tout ou partie des annexes cutanées, avantageusement sur les ongles, les cheveux, les poils, notamment les poils de barbe, des cils et/ou des sourcils, encore plus avantageusement sur les cheveux, plus préférentiellement les cheveux.

Le procédé de soin cosmétique permet par conséquent d'améliorer la couleur et/ou la brillance et/ou l'éclat et/ou le volume des annexes cutanées, avantageusement des cheveux.

Un autre objet concerne encore une méthode de traitement cosmétique pour augmenter la croissance des annexes cutanées et/ou diminuer leur chute, avantageusement des cheveux; et/ou pour maintenir et/ou augmenter les propriétés biomécaniques et/ou les propriétés de surface et/ou de texture des annexes cutanées, avantageusement des cheveux ; et/ou pour réparer les annexes cutanées abîmées, avantageusement les cheveux, et comprenant les étapes de :
- Identification d'une population d'humains dédiée ne souffrant pas d'une pathologie, notamment cutanée nécessitant un traitement thérapeutique, pour laquelle on souhaite appliquer l'hydrolysat d'*H*. *rhamnoides* ou une composition cosmétique le comprenant, et/ou qui en ont besoin ;
- Identification de la zone du corps saine et/ou de la peau saine, inclus le cuir chevelu sain, et/ou l'annexe cutanée saine nécessitant le traitement ;
- Application par voie topique de l'hydrolysat selon l'invention ou de la composition le comprenant, dans ce cas à une teneur comprise entre 1×10⁻⁴% à 10% en poids, préférentiellement de 1×10⁻⁴% à 5% en poids, encore avantageusement de 1×10⁻³% à 3% en poids, encore préférentiellement de 0,001% et 0,1 % en poids, par rapport au poids total de la composition.

Un dernier objet concerne un hydrolysat de tourteau d'*H*. *rhamnoides* pour son utilisation, seul ou dans une composition dermatologique ou pharmaceutique, laquelle comprend au moins un excipient dermatologiquement ou pharmaceutiquement acceptable, dans le traitement de l'alopécie et/ou de la calvitie. Dans un mode de réalisation avantageux de l'invention, l'hydrolysat est tel que décrit dans la présente invention et est présent dans la composition dermatologique ou pharmaceutique à une teneur comprise entre 1×10⁻⁴% à 10% en poids, préférentiellement de 1×10⁻⁴% à 5% en poids, encore avantageusement de 1×10⁻³% à 3% en poids, encore préférentiellement de 0,001% et 0,1 % en poids, par rapport au poids total de la composition.

Les exemples font partie intégrante de l'invention et toute caractéristique apparaissant nouvelle par rapport à un état de la technique antérieure à partir de la description prise dans son ensemble, incluant les exemples, fait partie intégrante de l'invention. Ainsi, chaque exemple a une portée générale.

Sauf indication contraire, la température est exprimée en degré Celsius et la pression est la pression atmosphérique.

### Exemples

### Exemple 1 : différents modes de préparation de l'hydrolysat de tourteau

Exemple 1a) : les protéines du tourteau ont été extraites durant une période d'une heure à température ambiante, c'est-à-dire à une température de 20°C, à pH 12, à partir d'une quantté de 10% en poids de tourteau par rapport au poids total de tourteau et d'eau comme solvant. Les protéines ainsi extraites ont été soumises à hydrolyse durant une période de 2 heures à une température de 55°C et à pH 8,5, avec une concentration en volume de 5% d'alcalase sous forme liquide par rapport au volume total d'alcalase et de protéines. L'enzyme a été inactivée par chauffage durant une période de 20 minutes à une température de 90°C et à pH 6,5. Le mélange a ensuite été refroidi, centrifugé, filtré (0,22µm).

Exemple 1b) : les protéines du tourteau ont été extraites durant une période d'une heure à température ambiante, c'est-à-dire à une température de 20°C, à pH 8,5, à partir d'une quantité de 10% en poids de tourteau par rapport au poids total de tourteau et d'eau comme solvant. Les protéines ainsi extraites ont été soumises à hydrolyse durant une période de 2 heures à une température de 55°C et à pH 8,5, avec une concentration en volume de 5% d'alcalase sous forme liquide par rapport au volume total d'alcalase et de protéines. L'enzyme a été inactivée par chauffage durant une période de 20 minutes à une température de 90°C et à pH 6,5. Le mélange a ensuite été refroidi, centrifugé, filtré (0,22µm).

Exemple 1c) : les protéines du tourteau ont été extraites durant une période d'une heure à température ambiante, c'est-à-dire à une température de 20°C, à pH 12, à partir d'une quantté de 10% en poids de tourteau par rapport au poids total de tourteau et d'eau comme solvant. Les protéines ainsi extraites ont été soumises à hydrolyse durant une période de 2 heures à une température de 55°C et à pH 8,5, avec une concentration en volume de 5% de papaïne sous forme liquide par rapport au volume total de papaïne et de protéines. L'enzyme a été inactivée par chauffage durant une période de 20 minutes à une température de 90°C et à pH 6,5. Le mélange a ensuite été refroidi, centrifugé, filtré (0,22µm).

### Exemple 2. Teneur en protéines totales de l'hydrolysat selon l'invention et analyse du profil de poids moléculaire peptidique

### Exemple 2a) Dosage des protéines totales :

*Matériel et méthode :* La teneur en protéines des matières premières et extraits a été estimée par dosage de l'azote total (méthode de Kjeldahl) et en multipliant la valeur obtenue par un facteur de 6,25 (N x 6,25).

*Résultats :* ils sont rassemblés dans le Tableau 1 (teneur en protéines des hydrolysats d'*H*. *rhamnoides).*

**[Tableau 1]**

| | Hydrolysat 1a) | Hydrolysat 1b) | Hydrolysat 1c) |
|---|---|---|---|
| Extrait sec (% en g/100g eau) | 3,84 | 3,44 | 2,72 |
| Teneur en protéines /MS (%) | 37,62 | 46,5 | 64,2 |
| Teneur en protéines dans l'hydrolysat natif (g/L) | 14,4 | 16,0 | 17,46 |
| Teneur en protéines dans l'hydrolysat concentré (g/L) | 18,81 | 23,25 | 32,1 |

| | | | |
|---|---|---|---|
| *Conclusion :* La teneur en protéines totales dans l'hydrolysat selon l'exemple 1a) est comprise entre 18 et 32 g/L de protéines (Tableau 1). Les protéines totales représentent entre 37 et 65 % de la matière sèche. | | | |

### Exemple 2b) Analyse du profil de poids moléculaire peptidique :

*Matériel et méthode :* La répartition des poids moléculaires des peptides de l'hydrolysat tel que préparé selon l'exemple 1a) a été analysée en perméation de gel sur colonne (Superose^{®} 12 10/300 GL, GE Healthcare Life Sciences). Les poids moléculaires ont été déterminés après étalonnage de la colonne par des molécules protéiques de poids moléculaire connu.

Résultats : ils sont rassemblés dans le Tableau 2.

**[Tableau 2]**

| % de peptides/total peptides | Hydrolysat 1a) | Hydrolysat 1a)' | Hydrolysat 1a)" |
|---|---|---|---|
| PM > 500kDa | 0,96 | 0,13 | 2,76 |
| 100<PM<500kDa | 4,14 | 0,3 | 0,13 |
| 30<PM<100kDa | 4,06 | 5,55 | 8,48 |
| 5kDa<PM<30kDa | 18,25 | 33,54 | 34,00 |
| PM<5kDa | 72,58 | 60,49 | 46,92 |

| | | | |
|---|---|---|---|
| *Conclusion :* l'hydrolysat tel que préparé selon l'exemple 1a) comprend plus de 70% de peptides de PM inférieur à 5kDa. | | | |

### Exemple 3. Effets in vitro de l'hydrolysat selon l'invention sur les propriétés biomécaniques des annexes cutanées

*Matériel et méthode :* Un réactif comprenant de l'albumine à 1,5%, du fer à 2 mM, de l'EDTA à 5 mM et de l'eau oxygénée H₂O₂ à 25 mM a été mis en présence ou non de l'hydrolysat d'*H*. *rhamnoides.* L'albumine s'oxyde en présence d'H₂O₂. La réaction d'oxydation est catalysée par le fer. Un contrôle de la catalyse de la réaction d'oxydation a été effectué en l'absence de fer. La mixture a été incubée à 37°C pendant 1 jour. La quantité de tryptophane, de dityrosine, de pentosidine et de produits de glycation a été mesurée par fluorescence (longueurs d'onde d'excitation/d'émission : 280 nm / 340 nm pour le tryptophane ; 315 nm / 410 nm pour la dityrosine ; 335 nm / 385 nm pour la pentosidine ; 370 nm / 440 nm pour les produits généraux de glycation).

*Résultats :* ils sont rassemblés dans les Tableaux 3 à 6.

### Exemple 3a) Diminution des produits de glycation (AGEs)

**[Tableau 3]**

| | Contrôle albumine et fer | Albumine et fer et hydrolysat d'*H*. *rhamnoides* selon ex. 1 a) (0,01% p/v) | Albumine et fer et hydrolysat d'*H*. *rhamnoides* selon ex. 1a) (0,1% p/v) |
|---|---|---|---|
| MOY (%) | 100 | 66,4 | 40,6 |
| ET | 5,5 | 1,5 | 2,8 |
| Ecart (%) vs contrôle | - | -33,6 | -59,4 |
| Statistique p-value vs contrôle | | <0,001 | <0,001 |

| | | | |
|---|---|---|---|
| *Conclusion :* La présence de fer catalyse le processus d'oxydation de l'albumine en présence de peroxyde d'hydrogène et s'accompagne de la formation de produits de glycation fluorescents. L'hydrolysat d'*H*. *rhamnoides à* 0,01% et 0,1% (p/v) a diminué la formation de ces produits de glycation de plus de 28% au moins. | | | |

### Exemple 3b) Diminution de la dégradation du tryptophane

**[Tableau 4]**

| | Contrôle albumine sans fer | Contrôle albumine avec fer | Albumine, fer et hydrolysat ex. 1a) 0,01% (p/v) | Albumine, fer et hydrolysat ex. 1a) 0,1% (p/v) |
|---|---|---|---|---|
| MOY (%) | 606,23 | 100,00 | 149,04 | 200,51 |
| ET (%) | 8,21 | 8,29 | 2,11 | 1,97 |
| Ecart (%) | | | 49 | 101 |
| Statistique p-value vs. contrôle (fer) | <0,001 | | <0,001 | <0,001 |

| | | | | |
|---|---|---|---|---|
| *Conclusion :* l'ajout de l'hydrolysat selon l'invention a diminué la dégradation du tryptophane d'au moins 30% suite à l'oxydation de l'albumine, montrant sa capacité à diminuer l'oxydation des protéines donc à maintenir et/ou augmenter les propriétés mécaniques des annexes cutanées, notamment des cheveux. | | | | |

### Exemple 3c) Diminution de la formation de dityrosine

**[Tableau 5]**

| | Contrôle albumine avec fer | Albumine, fer et hydrolysat ex. 1a) 0,01% (p/v) | Albumine, fer et hydrolysat ex. 1a) 0,1% (p/v) |
|---|---|---|---|
| MOY (%) | 100,00 | 59,98 | 38,49 |
| ET (%) | 3,91 | 1,12 | 0,54 |
| Ecart (%) | - | -40 | -62 |
| Statistique p-value vs. contrôle (fer) | | <0,001 | <0,001 |

| | | | |
|---|---|---|---|
| *Conclusion :* l'ajout de l'hydrolysat selon l'invention a diminué la formation de dityrosine d'au moins 35% suite à l'oxydation de l'albumine, montrant sa capacité à diminuer l'oxydation des protéines donc à maintenir et/ou augmenter les propriétés mécaniques des annexes cutanées, notamment des cheveux. | | | |

### Exemple 3d) Diminution de la formation de pentosidine

**[Tableau 6]**

| | Contrôle albumine avec fer | Albumine, fer et hydrolysat ex. 1a) 0,01% (p/v) | Albumine, fer et hydrolysat ex. 1a) 0,1% (p/v) |
|---|---|---|---|
| MOY (%) | 100,00 | 57,58 | 31,99 |
| ET (%) | 5,24 | 1,09 | 1,20 |
| Ecart (%) | - | -42 | -68 |
| Statistique p-value vs. contrôle (fer) | - | - | <0,05 |

| | | | |
|---|---|---|---|
| *Conclusion* : l'ajout de l'hydrolysat selon l'invention a diminué la formation de dityrosine d'au moins 35% suite à l'oxydation de l'albumine, montrant sa capacité à diminuer l'oxydation des protéines donc à maintenir et/ou augmenter les propriétés mécaniques des annexes cutanées, notamment des cheveux. | | | |

### Exemple 3e) Diminution d'un produit intermédiaire de glycation : les bases de Schiff

*Matériel et méthode :* L'hydrolysat tel que préparé selon l'exemple 1a) (0,01% ou 0,1% final (p/v)) a été ajouté ou non (Contrôle) à une solution tampon (phosphate buffer saline (PBS)) comprenant du cuivre (200µM) et des lipoprotéines (100µg/mL). Le mélange a été incubé durant une période de 48 heures à une température de 37°C. Laquantité (% moyen) de bases de Schiff a été mesurée par spectrométrie (Longueur d'onde d'excitation 370 nm / émission 440 nm).

### Résultat :

**[Tableau 7]**

| | Contrôle LDL + Cu₂₊ | LDL, cuivre, Hydrolysat selon l'ex. 1a) 0,01% (p/v) | LDL, cuivre, Hydrolysat selon l'ex. 1a) 0,1% (p/v) |
|---|---|---|---|
| MOY | 100 | 68,39 | 6,36 |
| ET (%) | 6,55 | 13,31 | 7,35 |
| p-value (Anova) vs. contrôle | | <0,05 | <0,05 |

| | | | |
|---|---|---|---|
| *Conclusion :* l'hydrolysat selon l'invention a diminué l'apparition de bases de Schiff d'au moins 11%. | | | |

### Exemple 4. Augmentation de la croissance des annexes cutanées

*Matériel et méthode :* Une suspension composée de fibroblastes de la papille de follicules pileux humains et de milieu de culture de base, contenant un cocktail de facteurs de croissance à 0,05% v/v et la présence ou non de l'hydrolysat d'*H*. *rhamnoides* à tester, a été centrifugée 5 minutes à 200 g pour former des agrégats. Des conditions contrôles ont été réalisées par l'ajout de cocktail de facteurs de croissance à 0,05 % v/v (Contrôle éthanol EtOH) et l'ajout de capsaïcine (10µm) en tant qu'agent sensibilisant des cellules (Contrôle Capsaïcine) dans le milieu de culture de base. Les agrégats ont été incubés à une température de 37°C sous atmosphère contrôlée (5% de CO et humidité relative de 95%).

Les agrégats ont été rincés dans une solution tamponnée saline (PBS) et les cellules ont été séparées par incubation dans une mixture de protéases (Collagénase A, Trypsine) avec de l'EDTA.

Une partie des cellules en suspension a été reprise dans du PBS pour l'analyse en cytométrie de flux. Le nombre de cellule et l'autofluorescence sont les deux paramètres mesurés. L'autofluorescence est un paramètre direct facilement mesurable utilisé comme marqueur de senescence cellulaire (Rattan *et al* 1982). Le nombre de cellule est un paramètre indirect du potentiel de croissance du follicule pileux. Une autre partie des cellules a été utilisée pour l'analyse du contenu des cellules en ADN et le taux de production d'ATP. Ces deux paramètres ont été mesurés selon les instructions des fournisseurs (Kit CyQuant NF Cell proliferation Assay C35006, Invitrogen et Kit Bioluminescence Assay Kit CLS II Roche 11699695001, Sigma Aldrich).

*Résultats* : ils sont rassemblés dans les tableaux 8 (effets de l'hydrolysat d'*H*. *rhamnoides* sur la quantité d'ADN des fibroblastes de la papille cultivés en agrégats) 9 (effets de l'hydrolysat d'*H*. *rhamnoides* sur la production d'ATP par les fibroblastes de la papille cultivés en agrégats), 10 (effets de l'hydrolysat d'*H*. *rhamnoides* sur le nombre de fibroblastes de la papille cultivés en agrégats, leur granularité et leur autofluorescence mesurés par cytométrie en flux).

**[Tableau 8]**

| | **MOY** | **% écart vs. témoin** | **P value (test Anova n=6)** |
|---|---|---|---|
| Contrôle EtOH | 100 | - | P<0,05 (T test) |
| Contrôle Capsaicine (10µM) | 23 | -77 | |
| Capsaïcine (10µM) + Hydrolysat ex. 1a) 0,1% (p/v) | 141 | +41 | P<0,05 |

| | | | |
|---|---|---|---|
| *Conclusion :* Le traitement des agrégats de fibroblastes de la papille sensibilisés par la capsaïcine à la concentration de 10 µM a réduit la quantité d'ADN dans les cellules. L'ajout conjoint de l'hydrolysat d'*H*. *rhamnoides à* 0,03% (p/v) a permis de réduire les impacts négatifs de la capsaïcine, montrant la capacité de l'hydrolysat à diminuer la chute des annexes cutanées, en particulier les cheveux. Le traitement des agrégats avec l'hydrolysat d'*H*. *rhamnoides à* 0,1% (p/v) a permis d'augmenter la quantité totale d'ADN (+41% par rapport au témoin), démontrant l'effet de l'hydrolysat selon l'invention sur l'augmentation de la croissance des annexes cutanées. | | | |

**[Tableau 9].**

| | **MOY** | **ET** | **% activité** | |
|---|---|---|---|---|
| Contrôle EtOH | 100 | 21 | - | P<0,05 (t-test) |
| Contrôle Capsaicine (10µM) | 24 | 3 | -76 | - |
| Capsaicine (10µM) + Hydrolysat ex.1a) 0,03% (p/v) | 154 | 17 | +54 | P<0,001 (t-test) |

| | | | | |
|---|---|---|---|---|
| *Conclusion :* Le traitement des agrégats de fibroblastes de la papille sensibilisés par la capsaïcine à la concentration de 10 µM a réduit la production d'ATP dans les cellules de 76% par rapport au contrôle (Ethanol). L'ajout conjoint à la capsaïcine de l'hydrolysat d'*H*. *rhamnoides* à 0,03% (p/v ) a permis de 1) contrecarrer les impacts négatifs de la capsaïcine, démontrant ainsi la capacité de l'hydrolysat selon l'invention à diminuer la chute des annexes cutanées, avantageusement des cheveux, 2) et de stimuler la production d'ATP d'au moins 16% par rapport au témoin non sensibilisé, montrant l'effet dudit hydrolysat sur l'augmentation de la croissance des annexes cutanées, préférentiellement des cheveux. | | | | |

**[Tableau 10]**

| | Nombre de cellules (%) | Granularité (%) | Autofluorescence (%) |
|---|---|---|---|
| Contrôle (EtOH) | 100 | 100 | 100 |
| Contrôle Capsaicine (10µm) | 28 | 217 | 235 |
| Capsaicine (10µm) + Hydrolysat ex. 1a) 0,03% (p/v) | 127 | 130 | 97 |

| | | | |
|---|---|---|---|
| *Conclusion :* Le traitement des agrégats de fibroblastes de la papille sensibilisés par la capsaïcine (10 µM) a réduit le nombre de fibroblastes de la papille, augmenté la granularité globale des cellules et augmenté la fluorescence par rapport au contrôle (EtOH), ceci impliquant la chute des cheveux. L'ajout conjoint à la capsaïcine de l'hydrolysat d'*H*. *rhamnoides à* 0,03% (p/v) a permis de réduire les impacts négatifs de la capsaïcine sur les trois paramètres mesurés et de stimuler le nombre de cellules de 54% par rapport au contrôle, montrant la capactié de l'hydrolysat à diminuer la chute des cheveux. En outre, le nombre de cellules mesuré en présence de l'hydrolysat est aussi plus important que le contrôle non sensibilisé à la capsaicine, montrant un effet d'augmentation du nombre de fibroblastes donc un effet sur la croissance des cheveux. Ces résultats ont montré le potentiel protecteur de l'hydrolysat d'*H*. *rhamnoides* contre la chute précoce ou induite des cheveux et un potentiel stimulateur de croissance capillaire. | | | |

### Exemple 5. Effet ex-vivo sur la croissance

*Matériel et méthode :* Des follicules humains ont été mis en culture *ex vivo* dans un milieu de croissance complet contenant 0,5 µg/ml d'insuline, et traité avec de la capsaïcine à 30 µM. Cette dernière molécule est utilisée comme agent provoquant le ralentissement de la croissance du follicule. Une condition contrôle sans traitement à la capsaïcine a été réalisée. Les follicules sensibilisés à la capsaïcine ont été traités ou non avec l'hydrolysat d'*H*. *rhamnoides à* 0,03% (p/v) . Chaque condition de culture a été évaluée sur au moins 10 follicules mis en culture isolément (n=10). Les follicules ont été incubés à 37°C en présence de CO₂ à 5 %. Le milieu de culture a été renouvelé 3 fois par semaine, durant environ 2 semaines. L'élongation des tiges pilaires des follicules en culture a été obtenue à partir de photographie prises à différent jour. La longueur des cheveux a été évaluée par analyse d'image (Image J) et présentée en µm sous forme d'une moyenne +/- erreur type.

*Résultats* : ils sont rassemblés dans le tableau 11 (Valeurs d'élongation du cheveu (µm))

**[Tableau 11]**

| Jour | Contrôle | Contrôle et capsaïcine (10µm) | Capsaïcine (10µm) et hydrolysat ex. 1a) 0,03% (p/v) | Statistique traité versus non traité (T-test) |
|---|---|---|---|---|
| | MOY (µm) | MOY (µm) | MOY (µm) | |
| 0 | 0 | 0 | 0 | >0,05 (non significatif) |
| 6 | 1278 | 806 | 899 | <0,05 |
| 8 | 1492 | 861 | 962 | <0,01 |
| 11 | 1628 | 869 | 1009 | <0,01 |
| 13 | 1722 | 938 | 1072 | <0,01 |
| 15 | 1721 | 936 | 1048 | <0,01 |

| | | | | |
|---|---|---|---|---|
| *Conclusion :* Les follicules pileux mis en culture ex vivo dans un milieu contenant 0,5 µg/ml d'insuline ont continué à croître, comme le témoigne l'élongation de la tige pilaire (Contrôle). Le traitement à la capsaïcine (30 µM) a provoqué une chute de l'élongation de manière visible dès le 6ème jour de culture par rapport au contrôle. Le traitement des follicules pileux, sensibilisés à la capsaïcine, avec l'hydrolysat d'*H*. *rhamnoides* à 0,03% (p/v) a permis d'améliorer l'élongation du cheveu de manière significative dès le 6ème jour de culture par comparaison aux follicules traités à la capsaïcine. | | | | |

### Exemple 6. Effet réparateur sur la fibre capillaire

*Matériel et méthode :* Des mèches de cheveux de couleur brun foncé de type caucasien ont été calibrées (1g ; 12 cm) et préparées pour l'étude. Les mèches de cheveux ont été lavées puis blanchies 3 fois à l'aide d'une solution de peroxyde d'hydrogène (5,6% H₂0₂ + 13,9% (NH4)₂S₂0₈, pH=9,4) durant une période de 30 minutes. Les mèches de cheveux saines (non blanchies) ont été conservées comme contrôle. Après lavage et séchage durant une période de 45 minutes à 55°C sous flux d'air, les mèches de cheveux ont été plongées durant 24 heures dans de l'eau distillée (comme contrôle) ou dans une solution aqueuse contenant 1% d'hydrolysat d'*H*. *rhamnoides.* Les deux solutions ont été préalablement tamponnées à pH 5,5. Les mèches ont été rincées puis séchées durant 1 heure à environ 60°C sous un flux d'air. La température de dénaturation des protéines humaines a été déterminée par calorimétrie différentielle (Wortmann *et al.* 1993) avec un taux de chauffe de 2 K/min et un analyseur enthalpique différentiel (DSC Q100, TA Instruments).

### Résultats :

**[Tableau 12]**

| | Cheveux sains non blanchis | Cheveux blanchis Contrôle | Cheveux blanchis + Hydrolysat ex. 1a) 1% (p/v) |
|---|---|---|---|
| Moyenne (°C) | 140,4 | 132 | 135,5 |
| ET (°C) | 0,07 | 0,09 | 0,21 |
| Ecart (°C) | 8,4 | - | 3,5 |
| Indice de réparation (%) | - | - | 41% |
| P value (test Anova n=6) | <0,001 | - | <0,001 |

| | | | |
|---|---|---|---|
| *Conclusion :* Les cheveux blanchis au peroxyde d'hydrogène sont plus instables que les cheveux sains non blanchis. La différence de température de dénaturation des protéines de la fibre capillaire a été de - 8°C entre les cheveux endommagés et les cheveux sans. La différence de température de dénaturation entre les cheveux peroxydés et traités avec un hydrolysat d'*H*. *rhamnoides* et les cheveux contrôle n'a été que de -4,9°C. Le traitement des cheveux endommagés a permis d'améliorer de +3,5°C la stabilité des protéines du cheveu face à la dénaturation par la chaleur. L'hydrolysat d'*H*. *rhamnoides* a montré un effet réparateur de la fibre capillaire endommagée par le peroxyde d'hydrogène. | | | |

### Exemple 7 : Exemples de compositions comprenant l'hydrolysat selon l'invention

### Exemple 7a) : shampoing

**[Tableau 13]**

| Phase | Dénomination | Quantité (% en poids total) |
|---|---|---|
| A | Eau | 52,95 |
| A | Cocamidopropyl betaïne | 9,46 |
| A | Coco-glucoside | 13,44 |
| A | Disodium laureth sulfosuccinate | 16,25 |
| A | Dicaprylyl ether, Lauryl alcool | 0,50 |
| A | Polyquaternium-7 | 1,0 |
| A | Benzoate de sodium | 0,50 |
| B | Eau | Qsp 3,10 |
| B | Hydrolysat selon l'exemple 1a) | 0,1-2 |
| C | Parfum | 0,05 |
| D | Polyéthylèneglycol/PPG-120/10 Trimethylolpropane | 1,50 |
| E | Acide citrique | 1,25 |

Le shampoing est préparé par les méthodes usuelles dans le domaine bien connu de l'homme du métier, en mélangeant les 4 phases et en ajustant la composition à un pH de 5,2 et à la viscosité de 2200 mPas (mesurée avec un appareil Brookfield (RVT ; 23°C, spindle 5 ; 50 tours par min).

### Exemple 7b: solution hydroalcoolique pour cuir chevelu

**[Tableau 14]**

| Phase | Dénomination | Quantité (% en poids total) |
|---|---|---|
| A | Eau | 84,35 |
| A | Chlorphénésine et méthylparabene | 0,30 |
| A | Gomme de xanthane | 0,10 |
| B | Ethanol à 96% | 10,00 |
| C | Eau | Qsp 3.25 |
| C | Hydrolysat selon l'exemple 1a) | 1-2 |

La solution hydroalcoolique est préparée par les méthodes usuelles dans le domaine bien connu de l'homme du métier, en mélangeant les 3 phases et en ajustant la composition à un pH de 6,2.

### Exemple 7c: masque pour cuir chevelu

**[Tableau 15]**

| Phase | Dénomination | Quantité (% en poids total) |
|---|---|---|
| A | Polyquaternium-37, carbonate de dicaprylyle, lauryle glucoside | 2,00 |
| A | Distéaroyléthyle hydroxyéthylmonium, méthosulfate, cétéaryle alcool | 1,00 |
| A | Glycéride végétales hydrogénés | 2,50 |
| A | cétéaryle alcool | 3,00 |
| A | carbonate de dicaprylyle | 0,50 |
| B | Eau | 87,05 |
| B | Benzoate de sodium | 0,40 |
| C | Hydrolysat selon l'exemple 1a) | 2 |
| C | Eau | 1.25 |
| D | Parfum | 0,5 |
| D | Acide citrique | q.s. |

Le masque est préparé par les méthodes usuelles bien connu de l'homme du métier, en mélangeant les 4 phases et en ajustant la composition à un pH de 4,1 et à la viscosité de 26 000 mPas (mesurée avec un appareil Brookfield (RVT ; 23°C, spindle 5 ; 50 tours par min).

### Exemple 7d: Sérum pour cheveux

**[Tableau 16]**

| Phase | Dénomination | Quantité (% en poids total) |
|---|---|---|
| A | Eau | 93,05 |
| A | Propylène glycol, phénoxyéthanol, chlorphénésine, méthylparabène | 2,50 |
| A | Glycérine | 1,00 |
| B | Gomme de xanthane | 0,20 |
| B | Polyacrylate de sodium | 0,25 |
| C | Huile de casto hydrogénée, Coceth-7, PPG-1 PEG-9 éther de lauryl glycol | 1,00 |
| C | Hydrolysat selon l'exemple 1a) | 2 |

## Revendications

1. Utilisation cosmétique non thérapeutique d'un hydrolysat de tourteau d'*Hippophae rhamnoides* pour augmenter la croissance des annexes cutanées et/ou diminuer leur chute, avantageusement des cheveux ; et/ou pour maintenir et/ou augmenter les propriétés biomécaniques et/ou les propriétés de surface et/ou de texture des annexes cutanées, avantageusement des cheveux ; et/ou pour réparer les annexes cutanées abîmées, avantageusement les cheveux.

2. Utilisation selon la revendication 1, **caractérisée en ce que** l'hydrolysat est un hydrolysat du coproduit d'extraction des graines d'*Hippophae rhamnoides* au CO₂ supercritique ou du coproduit obtenu par pressage desdites graines.

3. Utilisation selon la revendication 1 ou 2, **caractérisée en ce que** l'hydrolysat est un hydrolysat enzymatique.

4. Utilisation selon l'une quelconque des revendications 1 à 3, **caractérisée en ce que** l'hydrolysat est obtenu par digestion enzymatique à pH alcalin, avantageusement à un pH de 8,5.

5. Utilisation selon l'une quelconque des revendications 1 à 4, **caractérisée en ce que** l'hydrolysat contient :
- une teneur en matière sèche de 2 à 10% en poids,
- une teneur en protéines totales de 15 g/L à 35 g/L d'hydrolysat, avantageusement de 18,8g/L d'hydrolysat,
- un pourcentage en peptides de poids moléculaire de 5kDa à 30kDa compris entre 15 et 40%, avantageusement de 18,25%,
- un pourcentage en peptides de poids moléculaire de moins de 5KDa compris entre 40% et 85%, avantageusement de 72,6%.

6. Utilisation selon l'une quelconque des revendications précédentes, **caractérisée en ce que** l'utilisation est par voie topique.

7. Utilisation selon l'une quelconque des revendications précédentes, **caractérisée en ce que** l'hydrolysat de tourteau diminue les effets négatifs du vieillissement des annexes cutanées, avantageusement des cheveux, et/ou de la peau, avantageusement du cuir chevelu, et/ou des muqueuses.

8. Utilisation selon l'une quelconque des revendications précédentes **caractérisée en ce que** l'hydrolysat de tourteau améliore la couleur et/ou la brillance et/ou l'éclat et/ou le volume des annexes cutanées, avantageusement des cheveux.

9. Utilisation selon l'une quelconque des revendications précédentes **caractérisée en ce que** l'hydrolysat de tourteau est compris dans une composition cosmétique, à une teneur comprise entre 1×10⁻⁴% à 10% en poids, préférentiellement de 1×10⁻⁴% à 5% en poids, encore avantageusement de 1×10⁻³% à 3% en poids, encore préférentiellement de 0,001% et 0,1 % en poids, par rapport au poids total de la composition.

10. Utilisation selon la revendication 9, **caractérisée en ce que** la composition cosmétique comprend au moins un excipient cosmétiquement acceptable et que cette composition est choisi parmi un sérum, une lotion, une crème, un shampoing, un après-shampoing, une huile, un lait, une pommade, une pâte, une mousse, une émulsion, un hydrogel, un gel douche, un masque, une laque, un spray, une cire, un mascara, un crayon de maquillage, un vernis, avantageusement sous forme d'un shampoing, après-shampoing ou d'une lotion.

11. Procédé de soin cosmétique non thérapeutique comprenant l'application par voie topique d'un hydrolysat de tourteau *d'Hippophae rhamnoides* ou d'une composition cosmétique le comprenant pour augmenter la croissance des annexes cutanées, et/ou diminuer leur chute, avantageusement des cheveux ; et/ou pour maintenir et/ou augmenter les propriétés biomécaniques et/ou les propriétés de surface et/ou de texture des annexes cutanées, avantageusement des cheveux ; et/ou pour réparer les annexes cutanées abîmées, avantageusement les cheveux.

12. Procédé de soin cosmétique selon la revendication 11, **caractérisé en ce que** l'hydrolysat ou la composition cosmétique le comprenant est appliqué par voie topique sur tout ou partie de la peau du corps et/ou du visage choisie parmi le cuir chevelu, les jambes, les cuisses, les bras, le ventre, le décolleté, le cou, tout ou partie du visage, le front, le menton, les lèvres, le contour des lèvres, le contour des yeux, la zone dite « en T » du visage, et avantageusement le cuir chevelu, et/ou sur tout ou partie des annexes cutanées, plus préférentiellement les cheveux.

13. Hydrolysat de tourteau *d'Hippophae rhamnoides* ou composition dermatologique ou pharmaceutique le comprenant pour son utilisation dermatologique ou pharmaceutique dans le traitement de l'alopécie et/ou de la calvitie.

14. Hydrolysat selon la revendication 13, **caractérisé en ce qu'**il est tel que défini dans l'une quelconque des revendications 2 à 5.

## Patentansprüche

1. Nicht-therapeutische kosmetische Verwendung eines Hydrolysats aus dem Extraktionsrückstand von *Hippophae rhamnoides,* zur Förderung des Wachstums der Hautanhangsgebilde und/oder Verringerung des Ausfallens derselben, vorzugsweise des Kopfhaars; und/oder zur Aufrechterhaltung und/oder Stärkung der biomechanischen Eigenschaften und/oder der Oberflächeneigenschaften und/oder der Textur der Hautanhangsgebilde, vorteilhafterweise des Kopfhaars; und/oder zur Wiederherstellung von geschädigten Hautanhangsgebilden, vorzugsweise des Kopfhaars handelt.

2. Verwendung nach Anspruch 1, **dadurch gekennzeichnet, dass** es sich bei dem Hydrolysat um ein Hydrolysat des Nebenprodukts der Extraktion von Samen von *Hippophae rhamnoides* mit überkritischem CO₂ oder um das Nebenprodukt handelt, welches beim Pressen dieser Samen erhalten wird.

3. Verwendung nach Anspruch 1 oder 2, **dadurch gekennzeichnet, dass** es sich bei dem Hydrolysat um ein enzymatisches Hydrolysat handelt.

4. Verwendung nach einem beliebigen der Ansprüche 1 bis 3, **dadurch gekennzeichnet, dass** das Hydrolysat durch enzymatischen Verdau bei alkalischem pH-Wert, vorzugsweise bei einem pH-Wert von 8,5, erhalten wird.

5. Verwendung nach einem beliebigen der Ansprüche 1 bis 4, **dadurch gekennzeichnet, dass** das Hydrolysat Folgendes enthält:
- einen Trockensubstanzgehalt von 2 bis 10 Gewichts-%,
- einen Gesamtproteingehalt von 15 g/l bis 35 g/l an Hydrolysat, vorteilhafterweise von 18,8 g/l an Hydrolysat,
- einen Prozentanteil an Peptiden mit einem Molekulargewicht von 5 kDa bis 30 kDa, der im Bereich von 15 bis 40 % liegt, vorteilhafterweise 18,25 % beträgt,
- einen Prozentanteil an Peptiden mit einem Molekulargewicht von weniger als 5KDa, der im Bereich von 40 bis 85 % liegt, vorteilhafterweise 72,6 % beträgt.

6. Verwendung nach einem beliebigen der vorhergehenden Ansprüche, **dadurch gekennzeichnet, dass** die Verwendung auf topischem Wege erfolgt.

7. Verwendung nach einem beliebigen der vorhergehenden Ansprüche, **dadurch gekennzeichnet, dass** das Hydrolysat des Extraktionsrückstands die negativen Auswirkungen der Alterung der Hautanhangsgebilde, vorteilhafterweise des Kopfhaars, und/oder der Haut, vorteilhafterweise der Kopfhaut, und/oder der Schleimhäute verringert.

8. Verwendung nach einem beliebigen der vorhergehenden Ansprüche, **dadurch gekennzeichnet, dass** das Hydrolysat des Extraktionsrückstands die Farbe und/oder den Glanz und/oder die Lichtreflexion und/oder das Volumen der Hautanhangsgebilde, vorzugsweise des Kopfhaars, verbessert.

9. Verwendung nach einem beliebigen der vorhergehenden Ansprüche, **dadurch gekennzeichnet, dass** das Hydrolysat des Extraktionsrückstands in einer kosmetischen Zusammensetzung enthalten ist, zu einem Gehalt im Bereich von 1 x 10⁻⁴ % bis 10 % nach Gewicht, vorzugsweise von 1 x 10⁻⁴ % bis 5 % nach Gewicht, noch vorteilhafterweise von 1 x 10⁻³ % bis 3 % nach Gewicht, noch stärker bevorzugt von 0,001 % bis 0,1 % nach Gewicht, bezogen auf das Gesamtgewicht der Zusammensetzung.

10. Verwendung nach Anspruch 9, **dadurch gekennzeichnet, dass** die kosmetische Zusammensetzung mindestens einen kosmetisch unbedenklichen Hilfsstoff umfasst und dass diese Zusammensetzung aus einem Serum, einer Lotion, einer Creme, einem Shampoo, einer Haarspülung, einem Öl, einer Milch, einer Salbe, einer Paste, einem Schaum, einer Emulsion, einem Hydrogel, einem Duschgel, einer Maske, einem Haarlack, einem Spray, einem Wachs, einer Mascara, einem Schminkstift, einem Nagellack ausgewählt ist, wobei sie vorteilhafterweise in Form eines Shampoos, einer Haarspülung oder einer Lotion vorliegt.

11. Nicht-therapeutisches kosmetisches Pflegeverfahren, welches das Aufbringen auf topischem Wege eines Hydrolysats aus dem Extraktionsrückstand von *Hippophae rhamnoides* oder einer kosmetischen Zusammensetzung umfasst, welche ein solches umfasst, zur Förderung des Wachstums der Hautanhangsgebilde und/oder Verringerung des Ausfallens derselben, vorzugsweise des Kopfhaars; und/oder zur Aufrechterhaltung und/oder Stärkung der biomechanischen Eigenschaften und/oder der Oberflächeneigenschaften und/oder der Textur der Hautanhangsgebilde, vorteilhafterweise des Kopfhaars; und/oder zur Wiederherstellung von geschädigten Hautanhangsgebilden, vorzugsweise des Kopfhaars handelt.

12. Kosmetisches Pflegeverfahren nach Anspruch 11, **dadurch gekennzeichnet, dass** das Hydrolysat oder die kosmetische Zusammensetzung, welche ein solches umfasst, auf topischem Wege auf die Gesamtheit oder auf einen Teilbereich der Haut des Körpers und/der des Gesichts aufgebracht wird, wobei dieser aus der Kopfhaut, den Beinen, den Oberschenkeln, den Armen, dem Bauch, dem Dekolleté, dem Hals, der Gesamtheit oder einem Teilbereich des Gesichts, der Stirn, dem Kinn, den Lippen, der Lippenkontur, der Augenkontur, der sogenannten "T-Zone" des Gesichts ausgewählt ist, wobei es sich vorteilhafterweise um die Kopfhaut und/oder die Gesamtheit oder einen Teilbereich der Hautanhangsgebilde, stärker bevorzugt des Kopfhaars, handelt.

13. Hydrolysat aus dem Extraktionsrückstand von *Hippophae rhamnoides* oder dermatologische oder pharmazeutische Zusammensetzung, welche ein solches umfasst, zur dermatologischen oder pharmazeutischen Verwendung desselben/derselben in der Behandlung von Haarausfall und/oder Glatzenbildung.

14. Hydrolysat nach Anspruch 13, **dadurch gekennzeichnet, dass** es der Begriffsbestimmung in einem beliebigen der Ansprüche 2 bis 5 entspricht.

## Claims

1. The nontherapeutic cosmetic use of a hydrolyzate of *Hippophae rhamnoides* cake for increasing the growth of the skin appendages and/or decreasing the loss thereof, advantageously of the hair; and/or for maintaining and/or increasing the biomechanical properties and/or the surface and/or textural properties of the skin appendages, advantageously of the hair; and/or for repairing damaged skin appendages, advantageously the hair.

2. The use according to claim 1, wherein the hydrolyzate is a hydrolyzate of the coproduct from the extraction of *Hippophae rhamnoides* seeds with supercritical CO₂ or of the coproduct obtained by pressing said seeds.

3. The use according to claim 1 or 2, wherein the hydrolyzate is an enzymatic hydrolyzate.

4. The use according to any one of claims 1 to 3, wherein the hydrolyzate is obtained by enzymatic digestion at alkaline pH, advantageously at a pH of 8.5.

5. The use according to any one of claims 1 to 4, wherein the hydrolyzate contains:
- a dry matter content of 2% to 10% by weight,
- a total protein content of from 15 g/L to 35 g/L of hydrolyzate, advantageously 18.8 g/L of hydrolyzate,
- a percentage of peptides with a molecular weight of from 5 kDa to 30 kDa of between 15% and 40%, advantageously 18.25%,
- a percentage of peptides with a molecular weight of less than 5 kDa of between 40% and 85%, advantageously 72.6%.

6. The use according to any one of the preceding claims, wherein the use is via the topical route.

7. The use according to any one of the preceding claims, wherein the cake hydrolyzate reduces the negative effects of ageing of the skin appendages, advantageously of the hair, and/or of the skin, advantageously of the scalp, and/or of the mucous membranes.

8. The use according to any one of the preceding claims, wherein the cake hydrolyzate improves the color and/or sheen and/or radiance and/or volume of the skin appendages, advantageously of the hair.

9. The use according to any one of the preceding claims, wherein the cake hydrolyzate is included in a cosmetic composition in a content of between 1×10⁻⁴ to 10% by weight, preferentially from 1×10⁻⁴% to 5% by weight, more advantageously from 1×10⁻³% to 3% by weight, more preferentially from 0.001% and 0.1% by weight, relative to the total weight of the composition.

10. The use according to claim 9, wherein the cosmetic composition comprises at least one cosmetically acceptable excipient and in that this composition is chosen from a serum, a lotion, a cream, a shampoo, a hair conditioner, an oil, a milk, an ointment, a paste, a foam, an emulsion, a hydrogel, a shower gel, a mask, a lacquer, a spray, a wax, a mascara, a makeup pencil or a varnish, advantageously in the form of a shampoo, a conditioner or a lotion.

11. A nontherapeutic cosmetic care process comprising the topical application of a hydrolyzate of *Hippophae rhamnoides* cake or of a cosmetic composition comprising same, for increasing the growth of the skin appendages and/or decreasing the loss thereof, advantageously of the hair; and/or for maintaining and/or increasing the biomechanical properties and/or the surface and/or textural properties of the skin appendages, advantageously of the hair; and/or for repairing damaged skin appendages, advantageously the hair.

12. The cosmetic care process according to claim 11, wherein the hydrolyzate or the cosmetic composition comprising same is applied topically to all or part of the skin of the body and/or face chosen from the scalp, the legs, the thighs, the arms, the midriff, the neckline, the neck, all or part of the face, the forehead, the chin, the lips, the contour of the lips, the contour of the eyes, the "T" area of the face, and advantageously the scalp, and/or to all or part of the skin appendages, more preferentially the hair.

13. A hydrolyzate of *Hippophae rhamnoides* cake or a dermatological or pharmaceutical composition comprising same, for its dermatological or pharmaceutical use in the treatment of alopecia and/or baldness.

14. The hydrolyzate according to claim 13, wherein it is as defined in any one of claims 2 to 5.
